(19) Europäisches Patentamt
European Patent Office
Office européen des brevets

(11) EP 4 730 443 A1

(12) **EUROPEAN PATENT APPLICATION**
published in accordance with Art. 153(4) EPC

(43) Date of publication:
**22.04.2026 Bulletin 2026/17**

(21) Application number: **24856759.6**

(22) Date of filing: **16.08.2024**

(51) International Patent Classification (IPC):
***H01M 4/66*** *(2006.01)* ***H01M 4/13*** *(2010.01)*
***H01M 4/139*** *(2010.01)* ***H01M 4/04*** *(2006.01)*
***H01M 10/052*** *(2010.01)* ***H01M 10/42*** *(2006.01)*
***H01M 4/62*** *(2006.01)* ***H01M 4/02*** *(2006.01)*

(52) Cooperative Patent Classification (CPC):
**C08G 61/12; H01M 4/02; H01M 4/04; H01M 4/13; H01M 4/139; H01M 4/62; H01M 4/66; H01M 10/052; H01M 10/42;** Y02E 60/10

(86) International application number:
**PCT/KR2024/012244**

(87) International publication number:
**WO 2025/042147 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 KR 20230108421**
**18.08.2023 KR 20230108457**
**31.08.2023 KR 20230115816**

(71) Applicants:
- **LG Chem, Ltd.**
**Seoul 07336 (KR)**
- **LG Energy Solution, Ltd.**
**Seoul 07335 (KR)**

(72) Inventors:
- **SONG, In Taek**
**Daejeon 34122 (KR)**
- **KANG, Joon Koo**
**Daejeon 34122 (KR)**
- **KOH, Jong Kwan**
**Daejeon 34122 (KR)**
- **YANG, Hee Myeong**
**Daejeon 34122 (KR)**
- **KIM, Ki Hwan**
**Daejeon 34122 (KR)**
- **CHOI, Hyun Ju**
**Daejeon 34122 (KR)**
- **CHO, Woo Hyung**
**Daejeon 34122 (KR)**
- **LEE, Seok Kyeong**
**Daejeon 34122 (KR)**
- **LEE, Yu Mi**
**Daejeon 34122 (KR)**
- **PARK, Eun Kyoung**
**Daejeon 34122 (KR)**

(74) Representative: **Plasseraud IP**
**104 Rue de Richelieu**
**CS92104**
**75080 Paris Cedex 02 (FR)**

# (54) POLYMER LAYER

(57) The present specification discloses a polymer layer, a production method thereof, and a use thereof. The polymer layer exhibits a so-called PTC (Positive Temperature Coefficient) effect at a required level at a required time, whereby it can be applied as a material that can secure stability in applications where stability problems due to abnormally high heat or flame are caused. The polymer layer can exhibit oxidation potential characteristics suitable for a use, and stably maintain such oxidation potential characteristics even under harsh environments. In the polymer layer, the PTC effect can rapidly appear at a desired level at a required time. The polymer layer has a flat surface, and thus has excellent resistance to scratches, and the like. The present specification discloses a production method and a use of the polymer layer.



Processed by Luminess, 75001 PARIS (FR)

(Cont. next page)

[Figure 1]

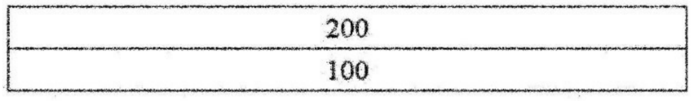

# Description

## Technical Field

This application claims the benefit of priority based on Korean Patent Application Nos. 10-2023-0108457 and 10-2023-0108421 dated August 18, 2023, and Korean Patent Application No. 10-2023-0115816 dated August 31, 2023, the disclosures of which are incorporated herein by reference in their entirety.

This specification discloses a polymer layer, a production method thereof, and a use thereof.

## Background Art

An energy storage technology has expanded application areas to mobile phones, tablet and notebook PCs, or electric vehicles, and the like.

As the data processing speed of mobile devices such as mobile phones or tablets increases and their used hours also increase, the development of secondary batteries with high energy density and operating potential, long cycle life, and low self-discharge rate is in progress.

As major developed countries suppress production of cars driven by internal combustion engines to alleviate global warming and air pollution, major automobile manufacturers also develop various electric vehicles, and thus the importance of secondary batteries having high energy density, high discharge voltage, and output stability increases as driving sources thereof.

According to the above trend, the occurrence frequency of ignition or explosion accidents due to overcharging, high-temperature exposure or outer shocks, and the like in devices or automobiles using secondary batteries as an energy source also increases.

As a main cause of such accidents, a short phenomenon, in which a positive electrode and a negative electrode inside an electrode assembly come into direct contact with each other mainly due to external stimuli, is known. When the secondary battery is overcharged or exposed to high temperatures or external stimuli, the short phenomenon can occur by the separator shrinkage due to the internal temperature increase of the secondary battery, or the internal structure destruction of the secondary battery due to outer shocks, and the like.

When the short phenomenon occurs, migration of lithium ions and electrons is concentrated through the region where the positive electrode and the negative electrode are in direct contact with each other, so that internal heat generation can be promoted. Accordingly, it is known that as gas or the like is generated inside the battery, the volume expands, and the risk of ignition increases.

## Disclosure

## Technical Problem

The present specification discloses a polymer layer, a production method thereof, and a use thereof. It is an object of the present specification to disclose a polymer layer applicable as a material capable of securing stability in applications where stability problems due to abnormally high heat or flame are caused by exhibiting a so-called PTC (Positive Temperature Coefficient) effect at a required level at a required time. It is another object of the present specification to disclose a polymer layer which exhibits oxidation potential characteristics suitable for applications and stably maintains such oxidation potential characteristics even under harsh environments. It is another object of the present specification to disclose a polymer layer in which the PTC effect rapidly appears at a desired level at a required time. It is another object of the present specification to disclose a polymer layer having a flat surface and thus having excellent resista nce to scratches, and the like. It is another object of the present specification to disclose a production method of the polymer layer and a use thereof.

## Technical Solution

In this specification, the term room temperature means a natural temperature without artificially warming or cooling. The room temperature may be, for example, any temperature within a range of 10°C to 30°C, or a temperature of about 23°C, about 25°C, or about 27°C or so.

Among physical properties mentioned in this specification, the physical property affected by the measurement temperature is a physical property measured at room temperature, unless otherwise specified.

The unit of temperature in this specification is Celsius (°C), unless otherwise specified.

In this specification, the term normal pressure means a natural pressure without artificially pressurizing or depressurizing, where a pressure within a range of about 730 mmHg to 790 mmHg or so may be generally regarded as the

normal pressure.

Among physical properties mentioned in this specification, the physical property affected by the measurement pressure is a physical property measured at normal pressure, unless otherwise specified.

In this specification, the standard-state humidity means any relative humidity within a range of 40% to 60%, where for example, a relative humidity of about 40%, 45%, 50%, 55%, or 60% may be referred to as the standard-state humidity.

Among physical properties mentioned in this specification, the physical property affected by the measurement humidity is a physical property measured at the standard-state humidity, unless otherwise specified.

In this specification, the term normal state means a normal operating state (e.g., a normal charging or discharging state of a secondary battery) and/or a storage state of an electrical/electronic device such as a secondary battery.

In this specification, the term abnormal state means a state where abnormal heating, ignition, and/or explosion occurs in an electrical/electronic device such as secondary batteries, or the risk of the abnormal heating, ignition, and/or explosion is increased. For example, a state where abnormal heating, ignition, or explosion occurs due to a short circuit phenomenon in a secondary battery, or a dangerous state where the possibility of the heating, ignition, or explosion is increased may be the abnormal state.

The present specification discloses a polymer layer.

The term polymer layer means a layer including a polymer. The lower limit of the polymer content in the polymer layer may be 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, or 95 wt% or so, and the upper limit thereof may be 100 wt%, 95 wt%, 90 wt%, 85 wt%, 80 wt%, 75 wt%, 70 wt%, 65 wt%, 60 wt%, 55 wt%, or 50 wt% or so. The content is the content of the polymer based on the total weight of the polymer layer. The content may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The polymer layer may not be a so-called electrode active material layer. The content of the electrode active material in the polymer layer may be controlled. For example, the upper limit of the content of the electrode active material in the polymer layer may be 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof may be 0 wt%. The content is the content of the electrode active material based on the total weight of the polymer layer. The content may be within a range of less than or equal to, or less than any upper limit arbitrarily selected from the above-listed upper limits; or within a range of more than or equal to, or more than the above-listed lower limit while being less than or equal to, or less than any upper limit arbitrarily selected from the above-listed upper limits.

The polymer layer may comprise a conductive polymer. As is known, the conductive polymer is a polymer exhibiting conductivity by a conjugated system of a polymer chain and/or doping, and the like. For example, the conductive polymer is a polymer exhibiting low resistance in a doping state and high resistance in a de-doping state, which may be a polymer designed so that the conversion between the doping and de-doping states may occur rapidly at a necessary time point in response to temperature and/or voltage.

The polymer layer may need to exhibit an appropriate level of oxidation potential depending on the application. For example, when the polymer layer is positioned between the current collector body and the active material layer, as described below, the polymer layer may need to exhibit a lower oxidation potential than the active material layer, and it is necessary for the low oxidation potential to be maintained even upon repeated charging/discharging and/or high-speed charging/discharging of the secondary battery. Otherwise, a potential drop phenomenon may occur during the process of the repeated charging/discharging and/or high-speed charging/discharging.

For example, in the polymer layer, the absolute value of $\Delta V$ of Equation 1 below may be adjusted.

[Equation 1]

$$\Delta V = 100\times(V_f - V_i)/V_i$$

In Equation 1, $V_i$ is the oxidation potential of the polymer layer after one cyclic voltammetry scan. The cyclic voltammetry scan may be performed at a scan rate of 0.83 mV/sec in a range of 3V to 4.5V, and the oxidation potential is an oxidation potential based on $Li/Li^+$.

In Equation 1, $V_f$ is the oxidation potential of the polymer layer after 10 cyclic voltammetry scans. The cyclic voltammetry scan may be performed at a scan rate of 0.83 mV/sec in a range of 3V to 4.5V, and the oxidation potential is an oxidation potential based on $Li/Li^+$.

The cyclic voltammetry scan is performed on a coin cell to which the polymer layer is applied.

$V_i$ and $V_f$ in Equation 1 have the same unit, and for example, the unit is V.

$V_i$ and $V_f$ in Equation 1 are measured in the manner described in "4. Measurement of oxidation potentials $V_i$ and $V_f$" of Example sections of this specification.

The upper limit of the absolute value of the $\Delta V$ may be 3%, 2.9%, 2.8%, 2.7%, 2.6%, 2.5%, 2.4%, 2.3%, 2.2%, 2.1%, 2.0%, 1.9%, 1.8%, 1.7%, 1.6%, 1.5%, 1.4%, 1.3%, 1.2%, 1.1%, 1.0%, 0.9%, 0.8%, 0.7%, or 0.6% or so, and the lower limit thereof may be, for example, 0%, 0.1%, 0.2%, 0.3%, 0.4%, 0.5%, 0.6%, 0.7, or 0.8% or so. The $\Delta V$ may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

The $\Delta V$ may be a positive number or a negative number.

It means that as the $\Delta V$ has a lower value, the polymer layer stably maintains a low oxidation potential.

The oxidation potential of the polymer layer may be adjusted to an appropriate level. In this instance, the oxidation potential is equal to $V_i$ of Equation 1 above.

The upper limit of the oxidation potential of the polymer layer may be 4.0 V, 3.95 V, 3.9 V, 3.85 V, 3.8 V, 3.75 V, 3.7 V, 3.65 V, 3.6 V, 3.55 V, 3.5 V, 3.45 V, or 3.4 V or so, and the lower limit thereof may be, for example, 1 V, 1.5 V, 2 V, 2.5 V, 3 V, 3.1 V, 3.2 V, 3.3 V, or 3.4 V or so. The oxidation potential may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

As described above, the conductive polymer included in the polymer layer may be a polymer exhibiting different electrical resistances in a doping state and a de-doping state, and simultaneously a polymer in which conversion between the doping and de-doping states rapidly appears at a necessary time point.

For example, in the polymer layer, Q in Equation 2 below may be adjusted.

[Equation 2]

$$Q = R_{3V}/R_{3.3V}$$

In Equation 2, $R_{3V}$ is the AC impedance resistance of the polymer layer under external voltage conditions of 25°C and 3V

In Equation 2, $R_{3.3V}$ is the AC impedance resistance of the polymer layer at the time point when 1 second has passed after the external voltage has been converted to 3.3V for the polymer layer exposed to the external voltage conditions of 25°C and 3V.

The resistances $R_{3V}$ and $R_{3.3V}$ in Equation 2 have the same unit, and for example, the unit is $\Omega$.

The resistances $R_{3V}$ and $R_{3.3V}$ in Equation 2 may be confirmed in the coin cell to which the polymer layer is applied. The resistances $R_{3V}$ and $R_{3.3V}$ in Equation 2 may be confirmed according to the manner described in "5. AC impedance resistance at 3V and 3.3V" of Example sections of this specification.

The lower limit of the Q value may be 10, 50, 70, 80, 90, 100, 110, 120, 130, or 140 or so, and the upper limit thereof may be 400, 350, 300, 250, 200, 150, or 100 or so. The Q value may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

It means that the higher the Q value of Equation 2 above, the faster the decrease in the resistance of the polymer layer occurs upon change of the external voltage conditions (from 3V to 3.3V).

The upper limit of the AC impedance resistance $R_{3.3V}$ of Equation 2 may be 5000, 4500, 4000, 3500, 3000, 2500, 2000, 1500, 1000, 900, 800, 700, 600, 500, 400, 350, 300, or 250 or so, and the lower limit thereof may be, for example, 10, 15, 20, 25, 30, 35, 40, 45, 50, 55, 60, 65, 70, 75, 80, 85, 90, 95, 100, 150, 200, 250, 300, or 400 or so. The unit of the $R_{3.3V}$ is $\Omega$. The $R_{3.3V}$ may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

The polymer layer may exhibit a high PTC effect.

For example, the polymer layer may be designed such that P in Equation 3 is within a predetermined range.

[Equation 3]

$$P = R_{130}/R_{25}$$

In Equation 3, $R_{130}$ is the AC impedance resistance of the polymer layer at 130°C, and $R_{25}$ is the AC impedance resistance of the polymer layer at 25°C.

In Equation 3, $R_{130}$ and $R_{25}$ have the same unit, and for example, the unit is Ω.

The AC impedance resistances $R_{130}$ and $R_{25}$ are resistances confirmed in the coin cell to which the polymer layer is applied, and the measurement method thereof is described in the "6. AC impedance resistance at room temperature (25°C) and 130°C" section of Example sections of this specification.

The lower limit of P in Equation 3 may be 60, 70, 80, 90 100, 150, 200, 250, 300, 350, 400, 450, 500, 550, 600, 650, 700, 750, 800, 850, 900, 950, 1,000, 1,100, 1,200, 1,300, 1,400 or 1,450 or so, and the upper limit thereof may be 10,000, 9,000, 8,000, 7,000, 6,000, 5,000, 4,000, 3,000, 2,000 1,500, 1,400, It may be 1,300, 1,200, 1,100, 1,000, 950, 900, or 850 or so. P in Equation 3 may be within a range of more than or equal to, or more than any lower limit arbitrarily selected from the above-listed lower limits; or within a range of less than, or equal to or less than any upper limit arbitrarily selected from the above-listed upper limits while being more than or equal to, or more than any lower limit arbitrarily selected from the above-listed lower limits.

The polymer layer may exhibit low resistance at room temperature (about 25°C) while exhibiting the P value.

For example, the upper limit of the resistance $R_{25}$ of the AC impedance of Equation 3 may be 500, 450, 400, 350, 300, 250, 200, 150, 100, 90, 80, 70, 60, 50, 45, 40, 35, 30, 28, 26, 24, or 22 or so, and the lower limit thereof may be, for example, 10, 15, 20, 25, 30, 35, or 40 or so. The unit of $R_{25}$ is Ω. The $R_{25}$ may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

The polymer layer may have a flat surface. For example, the upper limit of the arithmetic mean roughness Ra of the surface of the polymer layer may be 200, 190, 180, 170, 160, 150, 145, or 145 or so, and the lower limit thereof may be 10, 50, 100, 120, 140, or 160 or so. The unit of Ra is nm. The Ra may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits. The lower the Ra, the better the resistance to scratches, and the like may be secured. The Ra may be measured in the manner described in "8. Arithmetic mean roughness Ra" of Example sections of this specification.

In another example, the upper limit of the ratio Ra/T of the arithmetic mean roughness Ra of the polymer layer to the thickness T of the polymer layer may be 0.5, 0.45, 0.4, or 0.35 or so, and the lower limit thereof may be 0, 0.5, 1, 1.5, 2, 2.5, 3, or 3.5 or so. The Ra/T may be within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits. The lower the Ra/T, the better resistance to scratches, and the like may be secured. The Ra may be measured in the manner described in "8. Arithmetic mean roughness Ra" of Example sections of this specification, and the thickness T may be measured in the manner described in "3. Thickness measurement" of Example sections of this specification. The units of Ra and T are each nm.

Here, the thickness T of the polymer layer may be appropriately controlled according to the purpose. For example, the lower limit of the thickness T of the polymer layer may be 10 nm, 50 nm, 100 nm, 150 nm, 200 nm, 250 nm, 300 nm, 350 nm, or 400 nm or so, and the upper limit thereof may be 2 μm, 1.5 μm, 1 μm, 950 nm, 900 nm, 850 nm, 800 nm, 750 nm, 700 nm, 650 nm, 600 nm, 550 nm, 500 nm, 450 nm, or 400 nm or so. The thickness may be within a range of less than or equal to, or less than any upper limit arbitrarily selected from the above-listed upper limits while being more than or equal to, or more than any lower limit arbitrarily selected from the above-listed lower limits.

The conductive polymer included in the polymer layer may be a thiophene polymer.

The term thiophene polymer means a polymer containing a thiophene monomer unit in a certain level or more.

For example, the lower limit of the ratio of the mole number of the thiophene monomer unit in the thiophene polymer may be 50 mol%, 55 mol%, 60 mol%, 65 mol%, 70 mol%, 75 mol%, or 80 mol% or so, and the upper limit thereof may be 100 mol%, 95 mol%, 90 mol%, or 80 mol% or so. The ratio of the mole number of the thiophene monomer unit is the ratio ($100\times M_T/M$) of the mole number ($M_T$) of all thiophene monomer units present in the thiophene polymer or the mole number (MT) of all thiophene monomers applied to prepare the thiophene polymer to the mole number (M) of all monomer units present in the thiophene polymer or the mole number (M) of all monomers applied to prepare the thiophene polymer. The ratio ($100\times M_T/M$) of the thiophene monomer unit may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

The term monomer unit means a form in which a certain monomer is polymerized and included in a polymer molecule (polymer). The term thiophene monomer is a thiophene-based monomer, which means a monomer containing a thiophene skeleton.

The conductive polymer may contain a long-chain hydrocarbon functional group or a monomer unit (hereinafter, referred to as unit A) having the long-chain hydrocarbon functional group. The unit A may be a thiophene monomer unit.

The term long-chain hydrocarbon functional group means a monovalent hydrocarbon group having a carbon number in a certain number or more, or a monovalent functional group containing the monovalent hydrocarbon group.

For example, the lower limit of the carbon number (i.e., the carbon number of the monovalent hydrocarbon group) present in the long-chain hydrocarbon functional group may be 3, 4, 5, 6, 7, 8, 9, 10, 11, or 12 or so, and the upper limit thereof may be 30, 29, 28, 27, 26, 25, 24, 23, 22, 21, 20, 19, 18, 17, 16, 15, 14, 13, 12, 11, 10, 9, 8, 7, 6, 5, or 4 or so. The carbon number may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The carbon number may be the total carbon number present in the long-chain hydrocarbon functional group, or the carbon number of the hydrocarbon chain of the linear structure of the functional group. That is, the monovalent hydrocarbon group present in the long-chain hydrocarbon functional group may have a linear or branched structure. When the monovalent hydrocarbon group has the linear structure, the carbon number of the linear structure may be within the range. When the monovalent hydrocarbon group has the branched structure, the carbon number constituting the longest linear chain in the relevant branched structure may be within the range. For example, if the branched structure is a 2-ethylhexyl group, the carbon number constituting the longest linear chain is 6.

An example of the long-chain hydrocarbon functional group may be exemplified by one or more selected from the group consisting of an alkyl group, an alkenyl group, an alkynyl group, an alkoxy group, an alkylcarbonyl group, and an alkylcarbonyloxy group. In an appropriate example, the long-chain hydrocarbon functional group may be an alkyl group and/or an alkoxy group.

The carbon number present in the alkyl group, the alkenyl group, the alkynyl group, the alkoxy group, the alkyl group of the alkylcarbonyl group, and the alkyl group of the alkylcarbonyloxy group may be in the range of the carbon number (i.e., the carbon number of the monovalent hydrocarbon group) present in the long-chain hydrocarbon functional group.

The alkyl group, the alkenyl group, the alkynyl group, the alkoxy group, the alkyl group of the alkylcarbonyl group, and the alkyl group of the alkylcarbonyloxy group may be a linear or branched structure. In the case of a branched chain, the carbon number constituting the longest linear chain in the relevant branched structure may be in the range.

The alkyl group, the alkenyl group, the alkynyl group, the alkoxy group, the alkylcarbonyl group, or the alkylcarbonyloxy group, which is the long-chain hydrocarbon functional group, may also be optionally substituted with one or more substituents.

The long-chain hydrocarbon functional group may exhibit enhanced vibration energy at an increased temperature. This enhanced vibration energy may affect the doping and de-doping state of the conductive polymer. The degree of the vibration energy and the temperature at which the vibration energy appears are affected by the carbon number and arrangement state of the long-chain hydrocarbon functional group. Therefore, by adjusting the carbon number and amount of the long-chain hydrocarbon functional group and adjusting the arrangement state by the manufacturing method to be described below, it is possible to secure the characteristics of the polymer layer (for example, $\Delta V$, $V_f$ and $V_i$ of Equation 1 above, Q, $R_{3V}$ and $R_{3.3V}$ of Equation 2, $R_{130}$ and $R_{25}$ of Equation 3, etc.). For example, at the same temperature, the vibration energy of a relatively long chain is greater than that of a relatively short chain. Therefore, by appropriately employing a long chain and a short chain as the long-chain hydrocarbon functional group, it is possible to provide a conductive polymer to meet the intended effect.

Incidentally, the long-chain hydrocarbon functional group can provide appropriate mobility to a monomer or a polymer during the polymerization process of the conductive polymer, and accordingly, can also improve the polymerization efficiency.

For example, the ratio of the mole number ($M_L$) of the long-chain hydrocarbon functional group or the mole number ($M_L$) of the monomer unit (unit A) having the functional group to the mole number (M) of the total monomer units of the conductive polymer may be adjusted. For example, the lower limit of the ratio may be 20 mol%, 25 mol%, 30 mol%, 35 mol%, 40 mol%, 45 mol%, 50 mol%, 55 mol%, 60 mol%, 65 mol%, or 70 mol% or so, and the upper limit thereof may be 95 mol%, 90 mol%, 85 mol%, 80 mol%, or 75 mol% or so. The ratio is a ratio ($100\times M_L/M$) of the mole number ($M_L$) of all long-chain hydrocarbon functional groups present in the conductive polymer or the mole number ($M_L$) of monomer units having the long-chain hydrocarbon functional group or the mole number ($M_L$) of monomers having all long-chain hydrocarbon functional groups applied to prepare the thiophene polymer to the mole number (M) of all monomer units present in the conductive polymer or the mole number (M) of all monomers applied to prepare the conductive polymer. The ratio may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The conductive polymer may include a first hydrocarbon functional group and a second hydrocarbon functional group.

The first hydrocarbon functional group is a functional group having a relatively large carbon number among the

long-chain hydrocarbon functional groups. The lower limit of the carbon number of the first hydrocarbon functional group may be 10, 11, or 12 or so, and the upper limit thereof may also be 20, 19, 18, 17, 16, 15, 14, 13, 12, 11, or 10 or so. The carbon number of the first hydrocarbon functional group may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The second hydrocarbon functional group is a functional group having a relatively small carbon number among the long-chain hydrocarbon functional groups. The lower limit of the carbon number of the second hydrocarbon functional group may be 3, 4, 5, 6, 7, or 8 or so, and the upper limit thereof may also be 9, 8, 7, or 6 or so. The carbon number of the second hydrocarbon functional group may be within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The carbon number of each of the first and second hydrocarbon functional groups may be the carbon number of the hydrocarbon chain of the linear structure present in the hydrocarbon functional group. For example, the first and second hydrocarbon functional groups may each independently be one or more selected from the group consisting of an alkyl group, an alkenyl group, an alkynyl group, an alkoxy group, an alkylcarbonyl group, and an alkylcarbonyloxy group, and may be, in a suitable example, an alkyl group and/or an alkoxy group, where the carbon number may be the carbon number of the alkyl group, the alkenyl group, the alkynyl group, the alkoxy group, the alkyl group of the alkylcarbonyl group, and the alkyl group of the alkylcarbonyloxy group.

The alkyl group, the alkenyl group, the alkynyl group, the alkoxy group, the alkyl group of the alkylcarbonyl group, and the alkyl group of the alkylcarbonyloxy group may have a linear or branched structure, and in the case of the linear chain, the total carbon number may be in the range, and in the case of the branched chain, the carbon number constituting the longest linear chain in the branched structure may be in the range.

As described above, the carbon number of the long-chain hydrocarbon functional group is a functional group exhibiting enhanced vibration energy at an increased temperature, where the higher the carbon number at the same temperature, the higher the vibration energy. That is, the first hydrocarbon functional group exhibits higher vibration energy than the second hydrocarbon functional group at the same temperature, and the sum of the vibration energies of these two functional groups may optimize the properties of the conductive polymer that are difficult to precisely control with one functional group.

For example, the first hydrocarbon functional group exhibits enhanced vibration energy at an increased temperature, and the second hydrocarbon functional group dilutes the vibration energy by the first hydrocarbon functional group at the same temperature, whereby it is possible to precisely control the start temperature (onset point) of the de-doping of the conductive polymer according to the purpose.

The ratio of the sum mole number of the first and second hydrocarbon functional groups or the sum mole number of the monomer units having the first hydrocarbon functional group and the monomer units having the second hydrocarbon functional group relative to the mole number (M) of the total monomer units of the conductive polymer may adjusted in the same range as the ratio $100 \times M_L/M$ as described above.

The lower limit of the ratio ($M_2/M_1$) of the mole number ($M_2$) of the second hydrocarbon functional group or the mole number ($M_2$) of the monomer units having the second hydrocarbon functional group to the mole number ($M_1$) of the first hydrocarbon functional group or the mole number ($M_1$) of the monomer units having the first hydrocarbon functional group may be 0.01, 0.05, 0.1, or 0.5 or so, and the upper limit thereof may be 100, 95, 90, 85, 80, 75, 70, 65, 60, 55, 50, 45, 40, 35, 30, 25, 20, 15, 10, 9, 8, 7, 6, 5, 4, 3, 2, 1, or 0.7 or so. The ratio may be within a range of more than or equal to, or more than any lower limit arbitrarily selected from the above-listed lower limits while being less than or equal to, or less than any upper limit arbitrarily selected from the above-listed upper limits. The ratio $M_2/M_1$ may be changed in consideration of the level of the desired vibrational energy and the design value of the polymer layer.

The conductive polymer may contain a polar functional group together with the long-chain hydrocarbon functional group. The monomer having the polar functional group may be a thiophene monomer.

The term polar functional group is a functional group containing one, or two or more polar atoms, for example, oxygen and/or nitrogen. An example of such a functional group includes a carboxyl group, a hydroxyl group, an amino group, a cyano group, a nitro group, an ether group, an alkoxy group, and/or a functional group of Formula 1 below, but is not limited thereto.

Here, the alkoxy group may be, in one example, an alkoxy group with 1 to 20 carbon atoms, 1 to 16 carbon atoms, 1 to 12 carbon atoms, 1 to 8 carbon atoms or 1 to 4 carbon atoms. The alkoxy group may be linear, branched, or cyclic, and may be linear or branched, as appropriate. The alkoxy group may be optionally substituted with one or more substituents.

In one example, the polar functional group may be a functional group of Formula 1 below.

[Formula 1]

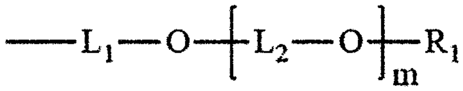

In Formula 1, $L_1$ is a single bond, an alkylene group or an alkylidene group, $L_2$ is an alkylene group or an alkylidene group, $R_1$ is hydrogen or an alkyl group, and m is an arbitrary number.

In Formula 1, the matter that $L_1$ is a single bond means a form that $L_1$ does not exist and the oxygen atom between $L_1$ and $L_2$ is directly connected to the monomer.

In one example, the alkyl group of $R_1$ in Formula 1 may be an alkyl group with 1 to 20 carbon atoms, 1 to 16 carbon atoms, 1 to 12 carbon atoms, 1 to 8 carbon atoms or 1 to 4 carbon atoms, or may be a methyl group or an ethyl group. The alkyl group may be linear, branched, or cyclic, and may be linear or branched, as appropriate. The alkyl group may be optionally substituted with one or more substituents.

The term alkylene group means a divalent functional group formed by removing one hydrogen atom from each of two different carbon atoms of an alkane, and the term alkylidene group means a divalent functional group formed by removing two hydrogen atoms from one carbon atom of an alkane.

In one example, the alkylene groups of $L_1$ and $L_2$ in Formula 1 may each be an alkylene group with 2 to 20 carbon atoms, 2 to 16 carbon atoms, 2 to 12 carbon atoms, 2 to 8 carbon atoms or 2 to 4 carbon atoms, or may each be an ethylene group or a propylene group. The alkylene group may be linear, branched, or cyclic, and may be linear or branched, as appropriate. The alkylene group may be optionally substituted with one or more substituents.

In one example, the alkylidene groups of $L_1$ and $L_2$ in Formula 1 may each be an alkylidene group with 1 to 20 carbon atoms, 1 to 16 carbon atoms, 1 to 12 carbon atoms, 1 to 8 carbon atoms or 1 to 4 carbon atoms, or may each be a methylidene group, an ethylene group or a propylidene group. The alkylidene group may be linear, branched, or cyclic, and may be linear or branched, as appropriate. The alkylidene group may be optionally substituted with one or more substituents.

In Formula 1, the lower limit of m may be 1, 2, 3, or 4, and the upper limit thereof may be 10, 9, 8, 7, 6, 5, 4, or 3 or so. The above n may be within a range of more than or equal to, or more than any lower limit arbitrarily selected from the above-listed lower limits while being less than or equal to, or less than any upper limit arbitrarily selected from the above-listed upper limits.

The polar functional group may bind the polymer layer to another layer to have an appropriate bonding force, and may significantly improve dispersibility of a conductive material, which is described below, in the polymer layer in combination with the long-chain hydrocarbon functional group. In addition, the polar functional group may also play a role in suppressing the PTC effect from appearing at a relatively low temperature.

The mole numbers of the polar functional group and the long-chain hydrocarbon functional group in the conductive polymer may be controlled to ensure an appropriate effect.

For example, the lower limit of the ratio ($M_L/M_L$) of the mole number ($M_L$) of the long-chain hydrocarbon functional group or the mole number ($M_L$) of the monomer units having the long-chain hydrocarbon functional group to the mole number ($M_L$) of the polar functional group or the mole number ($M_L$) of the monomer units having the polar functional group in the conductive polymer may be 1, 1.5, 2, 2.5, 3, 3.5, 4, 4.5, 5, 5.5, 6 6.5, 7, 7.5, 8, 8.5, or 9 or so, and the upper limit thereof may be 500, 450, 400, 350, 300, 250, 200, 150, 100, 90, 80, 70, 60, 50, 40, 30, 20, 19, 18, 17, 16, 15, 14, 13, 12, 11, 10, or 9.5 or so. The ratio $M_L/M_L$ may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits. Here, when the ratio $M_L/M_P$ is a ratio between monomer units, the relevant ratio may be a ratio of the mole number $M_L$ of monomer units having all long-chain hydrocarbon functional groups present in the conductive polymer to the mole number $M_P$ of monomer units having all polar functional groups; or a ratio of the mole number $M_L$ of monomers having all long-chain hydrocarbon functional groups applied to manufacture the conductive polymer to the mole number $M_P$ of monomers having all polar functional groups.

The ratio of the sum mole number $M_S$ of the monomer units having the long-chain hydrocarbon functional group and the monomer units having the polar functional group to the mole number M of all monomer units in the conductive polymer may be adjusted within the same range as the ratio $100 \times M_T/M$ as described above. In this instance, the mole number $M_S$ is substituted instead of the mole number $M_T$.

For example, the conductive polymer may contain a unit of Formula 2 below as the thiophene monomer unit.

[Formula 2]

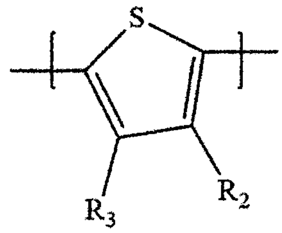

In Formula 2, $R_2$ and $R_3$ may each independently be hydrogen, the polar functional group, or the long-chain hydrocarbon functional group. In another example, $R_2$ and $R_3$ of Formula 2 may also be connected to each other to form a divalent functional group of Formula 3.

[Formula 3]

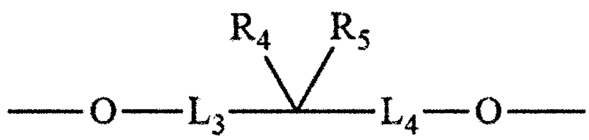

In Formula 3, $L_3$ and $L_4$ may each independently be a single bond, an alkylene group, or an alkylidene group, and $R_4$ and $R_5$ may each independently be hydrogen, the polar functional group, or the long-chain hydrocarbon functional group.

In Formula 2, when $R_2$ and $R_3$ are each independently hydrogen, a polar functional group, or a long-chain hydrocarbon functional group, at least one of $R_2$ and $R_3$ above may be the polar functional group or the long-chain hydrocarbon functional group.

In Formula 2, when $R_2$ and $R_3$ form the divalent functional group of Formula 3, at least one of $R_4$ and $R_5$ above may be the polar functional group or the long-chain hydrocarbon functional group.

In Formula 3, the meanings of the single bond, alkylene group, and alkylidene group, and specific examples are the same as in Formula 1. The technical significance and specific examples of the long-chain hydrocarbon functional group and polar functional group in Formulas 2 and 3 are as described above.

The ratio of the mole number $M_{C2}$ of the unit of Formula 2 to the mole number M of the total monomer units of the conductive polymer may be adjusted to be the same as the ratio $100 \times M_T/M$, where $M_T$ is replaced by $M_{C2}$.

In one example, the conductive polymer may contain a monomer unit represented by Formula 4 below. The monomer unit of Formula 4 above may be one example of the monomer units having the first hydrocarbon functional group.

[Formula 4]

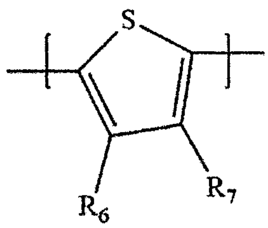

In Formula 4, $R_6$ and $R_7$ may each independently be hydrogen or the first hydrocarbon functional group. In this case, one or more of $R_6$ and $R_7$ above is the first hydrocarbon functional group.

In another example, $R_6$ and $R_7$ above may be connected to each other to form a divalent functional group of Formula 5.

[Formula 5]

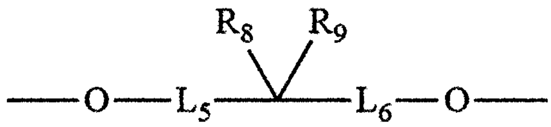

In Formula 5, $L_5$ and $L_6$ are each independently a single bond, an alkylene group, or an alkylidene group, and $R_8$ and $R_9$ are each independently hydrogen or the first hydrocarbon functional group, but at least one of $R_8$ and $R_9$ is the first hydrocarbon functional group.

The specific details of the first hydrocarbon functional group are as described above, and the specific details of the single bond, alkylene group, or alkylidene group are as described in Formula 1 above.

The conductive polymer may also contain a monomer unit represented by Formula 6 below. The monomer unit of Formula 6 above may be one example of the monomer units having the second hydrocarbon functional group.

[Formula 6]

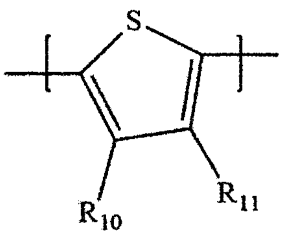

In Formula 6, $R_{10}$ and $R_{11}$ may each independently be hydrogen or the second hydrocarbon functional group, and in this case, one or more of $R_{10}$ and $R_{11}$ above is the second hydrocarbon functional group.

In another example, $R_{10}$ and $R_{11}$ above may be connected to each other to form a divalent functional group of Formula 7 below.

[Formula 7]

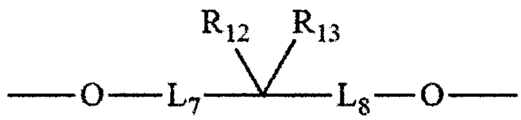

In Formula 7, $L_7$ and $L_8$ are each independently a single bond, an alkylene group, or an alkylidene group, and $R_{12}$ and $R_{13}$ are each independently hydrogen or the second hydrocarbon functional group, but at least one of $R_{12}$ and $R_{13}$ is the second hydrocarbon functional group.

The specific details of the second hydrocarbon functional group are as described above, and the specific details of the single bond, the alkylene group, and the alkylidene group are as described in Formula 1.

The conductive polymer may also contain a monomer unit represented by Formula 8 below. The monomer unit of Formula 8 above may be one example of the monomer units having the polar functional group.

[Formula 8]

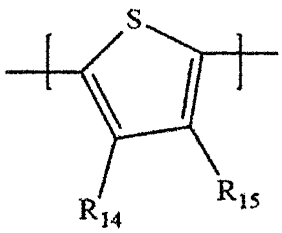

In Formula 8, $R_{14}$ and $R_{15}$ may each independently be hydrogen or the polar functional group. In the above case, one or more of $R_{14}$ and $R_{15}$ above are the polar functional group.

In another example, $R_{14}$ and $R_{15}$ of Formula 8 above may be connected to each other to form a divalent functional group of Formula 9 below.

[Formula 9]

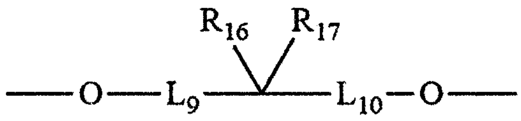

In Formula 9, $L_9$ and $L_{10}$ are each independently a single bond, an alkylene group, or an alkylidene group, and $R_{16}$ and $R_{17}$ are each independently hydrogen or a polar functional group, but at least one of $R_{16}$ and $R_{17}$ is the polar functional group.

The specific details of the polar functional group are as described above, and the specific details of the single bond, alkylene group, and alkylidene group are as described in Formula 1.

In the case where the conductive polymer simultaneously contains the monomer unit of Formula 4 above and the monomer unit of Formula 6 above, the ratio of the sum mole number $M_{4+6}$ of the monomer unit of Formula 4 above and the monomer unit of Formula 6 above to the mole number M of the total monomer units included in the conductive polymer may be adjusted within the same range as the ratio $100 \times M_L/M$ as described above, where $M_L$ is replaced by $M_{4+6}$.

The ratio of the mole number $M_4$ of the monomer unit of Formula 4 above and the mole number $M_6$ of the monomer unit of Formula 6 above may be adjusted within the same range as the ratio $M_2/M_1$. At this time, the mole number $M_1$ may be the mole number of the monomer unit of Formula 4 above, and the mole number $M_2$ may be the mole number of the monomer unit of Formula 6 above.

When the conductive polymer contains the monomer unit of Formula 8 above, the unit may be included so that the molar ratio $M_L/M_P$ is satisfied. In this instance, the mole number of the monomer unit of Formula 8 above becomes the mole number $M_P$. In addition, the mole number $M_L$ may be the mole number of the monomer unit of Formula 4 above, the mole number of the monomer unit of Formula 6 above, or the sum mole number of the monomer unit of Formula 4 above and the monomer unit of Formula 6 above.

When the monomer units of Formulas 4, 6, and 8 above are present in the conductive polymer, the ratio of the sum mole number $M_{4+6+8}$ of the monomer units of Formulas 4, 6, and 8 above to the sum mole number M of all monomer units in the conductive polymer may be adjusted within the same range as the ratio $100 \times M_T/M$. In this instance, the mole number $M_{4+6+8}$ becomes the mole number $M_T$.

The conductive polymer contains the monomer units, which may further contain other monomer units. For example, the conductive polymer may contain a no-thiophene monomer unit in addition to the thiophene monomer unit.

The no-thiophene monomer unit is a unit of a monomer other than a thiophene-based monomer, which is a unit for adjusting the oxidation potential of the conductive polymer. For example, the no-thiophene monomer unit may be a unit of a monomer which is copolymerizable with the thiophene monomer and exhibits a lower oxidation potential than polythiophene when manufactured as a homopolymer.

In the above case, the molar ratio of the thiophene monomer unit in the conductive polymer may be adjusted within the same range as the ratio $100 \times M_T/M$. In addition, the ratio of the no-thiophene monomer unit may be adjusted in consideration of the desired oxidation potential.

For example, in the conductive polymer, the lower limit of the mole number of the no-thiophene monomer unit per 1 mole of the thiophene monomer unit may be 0.05 mol, 0.1 mol, 0.15 mol, 0.2 mol, 0.22 mol, or 0.24 mol or so, and the upper limit thereof may be 1 mol, 0.9 mol, 0.8 mol, 0.7 mol, 0.6 mol, 0.5 mol, 0.4 mol, or 0.3 mol or so. The ratio may be within a range of less than or equal to, or less than any upper limit arbitrarily selected from the above-listed upper limits; or within a range of more than or equal to, or more than any lower limit arbitrarily selected from the above-listed lower limits; or within a range of more than or equal to, or more than any lower limit arbitrarily selected from the above-listed lower limits while being less than or equal to, or less than any upper limit arbitrarily selected from the above-listed upper limits.

The no-thiophene monomer unit may be, for example, an aromatic monomer unit or a nitrogen-containing heterocyclic monomer unit. The aromatic monomer unit is a unit formed from an aromatic monomer unit, and the nitrogen-containing heterocyclic monomer unit is a unit formed from a nitrogen-containing heterocyclic monomer.

For example, the no-thiophene monomer unit may be a monomer unit of Formula 10 below.

[Formula 10]

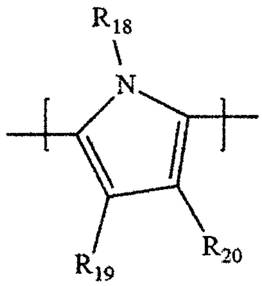

In Formula 10, $R_{18}$, $R_{19}$, and $R_{20}$ may each independently be hydrogen, the polar functional group, or the hydrocarbon functional group.

An example of the hydrocarbon functional group includes an alkyl group, an alkenyl group, or an aryl group.

The alkyl groups may each independently be an alkyl group with 1 to 20 carbon atoms, 1 to 16 carbon atoms, 1 to 12 carbon atoms, 1 to 8 carbon atoms, or 1 to 4 carbon atoms.

The alkenyl group may be an alkenyl group with 2 to 20 carbon atoms, 2 to 16 carbon atoms, 2 to 12 carbon atoms, 2 to 8 carbon atoms, or 2 to 4 carbon atoms.

The aryl group may be, for example, an aryl group with 6 to 30 carbon atoms, or 6 to 26 carbon atoms, or 6 to 22 carbon atoms, or 6 to 20 carbon atoms, or 6 to 18 carbon atoms, or 6 to 15 carbon atoms.

The alkyl group and the alkenyl group may each independently be linear, branched, or cyclic. The alkyl group, the alkenyl group, and the aryl group may each independently optionally be substituted with one or more substituents.

The specific details of the polar functional group are as described above. For example, the unit of Formula 10 above may be a polypyrrole unit.

In another example, the no-thiophene monomer unit may be a unit of Formula 11 below.

[Formula 11]

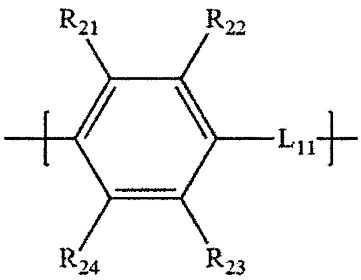

In Formula 11, $L_{11}$ may be a single bond, an alkylene group, an alkylidene group, O or $NR^1$. Here, the meaning of the single bond, the alkylene group or the alkylidene group is as described in Formula 1. In addition, the $R^1$ may be hydrogen, an alkyl group, an alkenyl group, or an aryl group.

In Formula 11, $R_{21}$ to $R_{24}$ may each independently be hydrogen, an alkyl group, an alkenyl group, an amino group, an alkoxy group, an aryl group, or a halogen atom.

The specific types of the alkyl group, the alkenyl group, and the aryl group in Formula 11 are as described in Formula 10.

The alkoxy group in Formula 11 may be an alkoxy group with 1 to 20 carbon atoms, 1 to 16 carbon atoms, 1 to 12 carbon atoms, 1 to 8 carbon atoms, or 1 to 4 carbon atoms. The alkoxy groups may each independently be linear, branched, or cyclic. The alkoxy group may be optionally substituted with one or more substituents.

For example, the monomer unit in Formula 11 may be a polyaniline or polyphenylene monomer unit.

In another example, the no-thiophene monomer unit may be a unit of Formula 12 below.

[Formula 12]

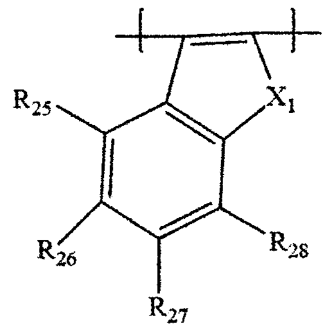

In Formula 12, $X_1$ may be S, O or $NR^1$. In addition, $R^1$ may be hydrogen, an alkyl group, an alkenyl group, or an aryl group.

In Formula 12, $R_{25}$ to $R_{28}$ may each independently be hydrogen, an alkyl group, an alkenyl group, an alkoxy group, an aryl group, or halogen.

The specific types of the alkyl group, the alkenyl group, the alkoxy group and the aryl group in Formula 12 are the same as in Formulas 10 and 11. The monomer unit of Formula 12 may be a polyindole unit.

In another example, the no-thiophene monomer unit may be a unit of Formula 13 below.

[Formula 13]

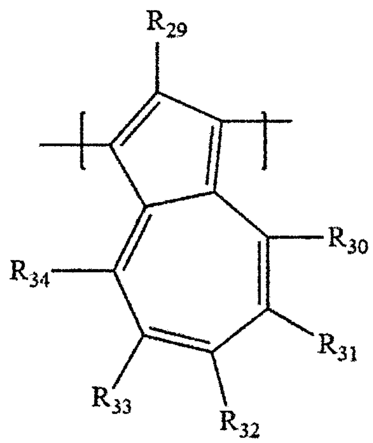

In Formula 13, $R_{29}$ to $R_{34}$ may each independently be hydrogen, an alkyl group, an alkenyl group, an alkoxy group, an aryl group, a hydroxy group, or halogen. The specific types of the alkyl group, alkenyl group, alkoxy group, and aryl group in Formula 13 are the same as in Formulas 10 and 11.

The monomer unit of Formula 13 above may be a polyazulene unit.

In another example, the no-thiophene monomer unit may be a unit of Formula 14 below.

[Formula 14]

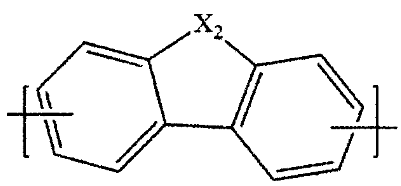

In Formula 14, $X_2$ may be a carbon atom ($CR^aR^b$) or a nitrogen atom ($NR^1$). Here, $R^a$, $R^b$, and $R^1$ may each independently be hydrogen, an alkyl group, an alkenyl group, or an aryl group.

Meanwhile, the unit of Formula 14 above may be unsubstituted or substituted with one or more selected from the group consisting of an alkyl group, an alkenyl group, an alkoxy group, an aryl group, and halogen.

The specific types of the alkyl group, the alkenyl group, the alkoxy group, and the aryl group in Formula 14 are the same as in Formulas 10 and 11.

For example, the monomer unit of Formula 14 above may be a polyfluorene or polycarbazole unit.

In another example, the no-thiophene monomer unit may be a unit of Formula 15 below.

[Formula 15]

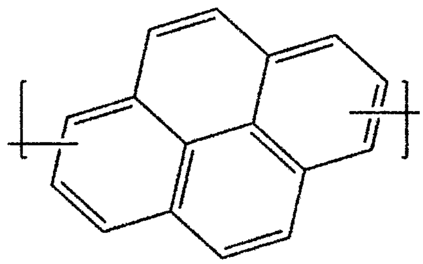

The unit of Formula 15 may be unsubstituted or substituted with one or more selected from the group consisting of an alkyl group, an alkenyl group, an alkoxy group, an aryl group, and halogen.

The specific types of the alkyl group, alkenyl group, alkoxy group and aryl group in Formula 15 above are the same as in Formulas 10 and 11. For example, the monomer unit of Formula 15 above may be a polypyrene unit.

The conductive polymer may have a weight average molecular weight in a predetermined range. The lower limit of the weight average molecular weight of the conductive polymer may be 10,000, 15,000, 20,000, 25,000, 30,000, 35,000, 40,000, 45,000, 50,000, 55,000, 60,000, 65,000, 70,000, 75,000, 80,000, 85,000, 90,000, or 95,000 or so, and the upper limit thereof may also be 1,000,000, 950,000, 900,000, 850,000, 800,000, 750,000, 700,000, 650,000, 600,000, 550,000, 500,000, 450,000, 400,000, 350,000, 300,000, 250,000, 200,000, 150,000, 130,000, 110,000, or 100,000 or so. The weight average molecular weight may be within a range of less than or equal to, or less than any upper limit arbitrarily selected from the above-listed upper limits while being more than or equal to, or more than any lower limit arbitrarily selected from the above-listed lower limits. The unit of the weight average molecular weight is g/mol, which may be confirmed according to the description of "2. GPC (Gel Permeation Chromatograph)" of Example sections of this specification.

The molecular weight distribution of the conductive polymer, i.e., the ratio (Mw/Mn) of the weight average molecular weight (Mw) to the number average molecular weight (Mn) may be within a predetermined range. The lower limit of the molecular weight distribution may be 2, 2.5, 3, or 3.5 or so, and the upper limit thereof may be 8, 7.5, 7, 6.5, 6, 5.5, 5, 4.5, 4, or 3.7 or so. The molecular weight distribution may have a range of less than or equal to, or less than any upper limit arbitrarily selected from the above-listed upper limits while being more than or equal to, or more than any lower limit arbitrarily selected from the above-listed lower limits. The molecular weight distribution may be confirmed according to the description of "2. GPC (Gel Permeation Chromatograph)" of Example sections of this specification.

The polymer layer may comprise a conductive material together with the conductive polymer. The conductive material may play a role in allowing the polymer layer to exhibit desired characteristics (e.g., $\Delta V$, $V_f$ and $V_i$ of Equation 1 above, Q, $R_{3V}$ and $R_{3.3V}$ of Equation 2, $R_{130}$ and $R_{25}$ of Equation 3, etc.) by being combined with the conductive polymer.

As the conductive material, a material having appropriate conductivity may be used. For example, as the conductive material one, or two or more selected from carbon particles, carbon fibers, graphene, graphite, carbon black, carbon nanotubes, and metal particles, and the like may be used.

As the conductive material, an appropriate type may be selected and used from the foregoing, and the shape of the material may be a particle shape (spherical, irregular, or other shape), a plate shape, or a fiber shape, and the like, but is not limited thereto.

The size of the conductive material may also be appropriately adjusted as needed. For example, the lower limit of the size of the conductive material may be 0.01 nm, 0.1 nm, 0.5 nm, 1 nm, 5 nm, 10 nm, 20 nm, 30 nm, 40 nm, 50 nm, or 60 nm or so, and the upper limit thereof may be 100000 nm, 90000 nm, 80000 nm, 70000 nm, 60000 nm, 50000 nm, 40000 nm, 30000 nm, 20000 nm, 10000 nm, 5000 nm, 1000 nm, 950 nm, 900 nm, 850 nm, 800 nm, 750 nm, 700 nm, 650 nm, 600 nm, 550 nm, 500 nm, 450 nm, 400 nm, 350 nm, 300 nm, 250 nm, 200 nm, 200 nm or 150 nm, 100 nm, 90 nm, 80 nm, 70 nm, or 65 nm o so. The size may be within a range of less than or equal to, or less than any upper limit arbitrarily selected from the above-listed upper limits while being more than or equal to, or more than any lower limit arbitrarily selected from the above-listed lower limits. The size is the average diameter (so-called D50 particle diameter) confirmed according to the manner described in "7. Average particle diameter" of Example sections of this specification.

Considering dispersibility, and the like, the conductive material may also be surface-treated. Such surface treatment is important in securing a dispersion state of the conductive material that can secure the desired characteristics (e.g., $\Delta V$, $V_f$ and $V_i$ of Equation 1 above, Q, $R_{3V}$ and $R_{3.3V}$ of Equation 2, $R_{130}$ and $R_{25}$ of Equation 3, Ra and Ra/T, etc.).

A surface-treating agent having appropriate compatibility with the conductive polymer may be used as the surface-treating agent for the surface treatment. For example, the conductive material may be surface-treated with a polyphenol-based compound as the surface-treating agent. The polyphenol-based compound means a compound including a structure containing two or more hydroxyl groups which are substituted in benzene, and connected thereto. Such a compound may be exemplified by a so-called catechol compound (i.e., catechol, or compound including the relevant structure), and an example thereof includes dopamine, polydopamine, 3,4-dihydroxy phenyl alanine, norepinephrine, tannic acid, humic acid, and/or lignin, and the like, but is not limited thereto.

The method of surface-treating the conductive material with the surface-treating agent is not limited, and for example, a method of mixing the conductive material and the surface-treating agent in an appropriate solvent, and the like, or a method of synthesizing or polymerizing the surface-treating agent on the surface of the conductive material may be applied.

When the conductive material is used, the ratio of the conductive material in the polymer layer may be adjusted depending on the purpose. For example, the lower limit of the weight ratio of the conductive material, relative to 100 parts by weight of the conductive polymer in the polymer layer, may be 1 part by weight, 5 parts by weight, 10 parts by weight, 15 parts by weight, 20 parts by weight, 25 parts by weight, 30 parts by weight, 35 parts by weight, 40 parts by weight, 42 parts by weight, 44 parts by weight, 46 parts by weight, 48 parts by weight, 50 parts by weight, 52 parts by weight, 54 parts by weight, 56 parts by weight, 58 parts by weight, 60 parts by weight, 65 parts by weight, 80 parts by weight, 90 parts by weight, or 95 parts by weight or so, and the upper limit thereof may be 1,000 parts by weight, 950 parts by weight, 900 parts by weight, 850 parts by weight, 800 parts by weight, 750 parts by weight, 700 parts by weight, 650 parts by weight, 600 parts by weight, 550 parts by weight, 500 parts by weight, 450 parts by weight, 400 parts by weight, 350 parts by weight, 300 parts by weight, 250 parts by weight, 200 parts by weight, 150 parts by weight, 145 parts by weight, 140 parts by weight, 135 parts by weight, 130 parts by weight, 125 parts by weight, 120 parts by weight, 115 parts by weight, 110 parts by weight, 105 parts by weight, 100 parts by weight, 95 parts by weight, 90 parts by weight, 85 parts by weight, 80 parts by weight, 75 parts by weight, 70 parts by weight, 65 parts by weight, 60 parts by weight, 55 parts by weight, or 50 parts by weight or so. The content may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

Under such a ratio, the conductive material may form a polymer layer of a desired shape effectively by appropriately interacting with the conductive polymer.

The polymer layer may also comprise any additional component if it comprises the conductive polymer and the conductive material.

The present specification discloses a method of forming the polymer layer.

As described above, in order that the polymer layer exhibits the desired effect (e.g., $\Delta V$, $V_f$ and $V_i$ of Equation 1 above, Q, $R_{3V}$ and $R_{3.3V}$ of Equation 2, $R_{130}$ and $R_{25}$ of Equation 3, Ra and Ra/T, etc.), selection of the long-chain hydrocarbon functional group and/or the conductive material is important, and additionally, control of the arrangement state of the long-chain hydrocarbon functional group and/or the dispersion state of the conductive material is important, where the desired arrangement and/or dispersion state may be secured by a manufacturing method to be described below.

The manufacturing method may comprise a step for forming a polymer layer with the above-described characteristics.

The manufacturing method may comprise, for example, a step of forming a polymer layer precursor including the conductive polymer and the conductive material, and a step of heat-treating the polymer layer precursor.

The conductive polymer, and the like may be produced by a known method. For example, as a method for producing polythiophene, a method using an oxidation polymerization reaction or a method using a radical reaction is known, and such a method may also be applied to the process of forming the conductive polymer. In addition, a commercially available product may be used as the conductive material, and its surface treatment may also be performed by a known method.

The polymer layer precursor is, for example, a layer including the conductive polymer and the conductive material, which means a layer converted into the polymer layer. Such a precursor may be formed by a known method, and may be formed, for example, by a method of coating a polymer solution in which the conductive polymer, or the like is dispersed in an appropriate solvent.

As the solvent, an appropriate solvent capable of dispersing the conductive polymer and the conductive material may be selected, and for example, ether-based solvents such as diethyl ether, tetrahydrofuran, dioxane, trioxane, dimethoxy ethane or toluene; aromatic hydrocarbon-based solvents such as ethyl benzene or alicyclic hydrocarbon solvents such as cyclohexane; tertiary amine-based solvents such as tetramethylethylenediamine (TMEDA) or hexamethylphosphorictriamide (HMPA), and the like may be used, or mixed solvents containing two or more of the foregoing, and the like may be used, without being limited thereto.

A polymer layer precursor is formed using the polymer solution. This process may be typically performed through a process of coating the polymer solution on an appropriate process base material. In this instance, the method of coating is not particularly limited.

The manufacturing method further comprises a step of heat-treating the precursor of the polymer layer. By adjusting the conditions in this process, the orientation state of the conductive polymer (e.g., the orientation state of the long-chain hydrocarbon functional group and/or the polar functional group) and/or the dispersion state of the conductive material is adjusted, whereby it is possible to form a polymer layer exhibiting the desired effect.

The heat treatment step may be performed in two steps. For example, the heat treatment step may comprise a first step of performing primary heat treatment on the polymer layer precursor; and a second step of performing secondary heat treatment on the polymer layer precursor undergone the first step.

The conditions of the first and second steps may be adjusted to achieve the desired orientation or alignment of the functional group and the dispersion state of the conductive material.

For example, the temperature $T_1$ of the primary heat treatment and the temperature $T_2$ of the secondary heat treatment may be adjusted. For example, the lower limit of the ratio ($T_1/T_2$) of the temperatures $T_1$ and $T_2$ may be 0.1, 0.2, 0.3, 0.4, 0.5, 0.6, 0.7, 0.8, 0.9, 1, 1.1, or 1.2 or so, and the upper limit thereof may be 10, 5, 4.5, 4, 3.5, 3, 2.5, 2, 1.9, 1.8, 1.7, 1.6, 1.5, 1.4, or 1.3, or 1.2 or so. The ratio may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

For example, the temperature $T_1$ of the primary heat treatment may be adjusted within a predetermined range. For example, the lower limit of the temperature $T_1$ may be 50°C, 55°C, 60°C, 65°C, 70°C, 75°C, 80°C, 85°C, 90°C, 95°C, 100°C, 105°C, 110°C, 115°C, 120°C, 125°C, 130°C, 135°C, or 140°C or so, and the upper limit thereof may be 300°C, 290°C, 280°C, 270°C, 260°C, 250°C, 240°C, 230°C, 220°C, 210°C, 200°C, 190°C, 180°C, 170°C, 160°C, 150°C, or 140°C or so. The temperature $T_1$ may be within a range of more than or equal to, or more than any lower limit arbitrarily selected from the above-listed lower limits; or within a range of less than or equal to, or less than any upper limit arbitrarily selected from the above-listed upper limits while being more than or equal to, or more than any lower limit arbitrarily selected from the above-listed lower limits.

The ratio of the heat treatment time $S_1$ in the primary heat treatment and the heat treatment time $S_2$ in the secondary heat treatment may be additionally adjusted. For example, the lower limit of the ratio $S_2/S_1$ may be 1, 2, 3, 4, 5, 6, 7, 8, 9, 10, 11, 12, 13, 14, 15, 20, 50, 70, 100, 110, 120, 130, 140, 150, 160, 170, 180, 190, 200, 210, 220, 230, 240, 250, 260, or 270 or so, and the upper limit thereof may be 1,000, 950, 900, 850, 800, 750, 700, 650, 600, 550, 500, 450, It may be 400, 350, 300, 290, 280, 270, 250, 200, 150, 100, 90, 80, 70, 60, 50, 40, 30, or 20 or so. The ratio $S_2/S_1$ may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

The lower limit of the secondary heat treatment time $S_2$ may be 0.1 hours, 0.2 hours, 0.3 hours, 0.4 hours, 0.5 hours, 0.6 hours, 0.7 hours, 0.8 hours, 0.9 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, or 18 hours or so, and the upper limit thereof may be 50 hours, 48 hours, 46 hours, 44 hours, 42 hours, 40 hours, 38 hours, 36 hours, 34 hours, 32 hours, 30 hours, 28 hours, 26 hours, 24 hours, 22 hours, 20 hours, or 18 hours. The secondary heat treatment time $S_2$ may be within a range of more than or equal to, or more than any lower limit arbitrarily selected from the above-listed lower limits; or within a range of less than or equal to, or less than any upper limit arbitrarily selected from the above-listed upper limits; or within a range of less than or equal to, or less than any upper limit arbitrarily selected from the above-listed upper limits while being more than or equal to, or more than any lower limit arbitrarily selected from the above-listed lower limits.

The polymer layer may be formed through the process.

The present specification discloses a current collector, an electrode, or an electrode assembly, comprising the polymer layer.

The current collector may comprise a current collector body and the polymer layer formed on the current collector body. The current collector may be used to form an electrode. For example, the electrode formed using the current collector may comprise the current collector and an active material layer formed on the polymer layer of the current collector. Figure 1 is a cross-sectional schematic diagram of a current collector comprising a current collector body (100) and a polymer layer (200), and Figure 2 is a diagram showing an electrode in which an active material layer (300) is formed on the polymer layer (200) of the current collector.

As shown in the diagram, the current collector body (100) and the polymer layer (200), and the polymer layer (200) and the active material layer (300) in the current collector or electrode may be in contact with each other, or other elements may also exist therebetween. In addition, although the drawing shows a case where the active material layer

(300) exists only on one side of the current collector body (100), the active material layer (300) may also exist on both sides of the current collector body (100). In this case, the polymer layer (200) may also exist in two layers between the respective active material layers (300) existing on both sides of the current collector body (100) and the current collector body (100), and may also exist in one layer between any one of the active material layers (300) existing on both sides and the current collector body (100).

The electrode formed by the current collector may be a negative electrode (anode) or a positive electrode (cathode) applied to a secondary battery.

The above-described polymer layer may be used as the polymer layer in the current collector and electrode. Such a polymer layer may also be formed by using the current collector body as a process base material in the process of forming the above-described polymer layer, and may also be formed in a manner of transferring the polymer layer formed through a separate process to the current collector body.

The excellent electrical properties, the adjusted PTC effect, and the oxidation potential of the polymer layer enable the electrode or the secondary battery to which the electrode is applied to be stably operated and stored in a normal state, and to secure stability in an abnormal state.

As the current collector body, one commonly used as a current collector body for a positive or negative electrode may be used without any special limitation.

If the current collector body has conductivity without causing a chemical change in an application device such as a secondary battery, its type, size, shape, and the like are not particularly limited. An example of a material usable as the current collector body may include copper, aluminum, stainless steel, nickel, titanium, or calcined carbon, and the like, or may be exemplified by materials surface-treated with carbon, nickel, titanium, or silver, and the like on the surface of copper, aluminum, or stainless steel. The current collector body may be in the form of a film, sheet, foil, net, porous body, foam, or non-woven fabric, including the material. In some cases, a known surface treatment may also be performed on the surface of the current collector body to improve adhesive force to other layers such as a polymer layer or an active material layer.

Such a current collector body may typically have a thickness within a range of 3 $\mu$m to 500 $\mu$m, but is not limited thereto.

A commonly applied layer may also be used as the active material layer used to form the electrode. Typically, the active material layer comprises an electrode active material. The specific type of the electrode active material is not particularly limited, and materials to form a positive electrode or a negative electrode may usually be used.

For example, when the active material layer is a positive electrode active material layer, the electrode active material may include layered compounds such as lithium cobalt oxide ($LiCoO_2$) or lithium nickel oxide ($LiNiO_2$), or compounds substituted with one or more transition metals; lithium iron oxides such as $LiFe_3O_4$; lithium manganese oxides such as Formula $Li_{1+c1}Mn_{2-c1}O_4$ ($0 \leq c1 \leq 0.33$), $LiMnO_3$, $LiMn_2O_3$, or $LiMnO_2$; lithium copper oxides ($Li_2CuO_2$); vanadium oxides such as $LiV_3O_8$, $V_2O_5$, or $Cu_2V_2O_7$; Ni-site lithium nickel oxides represented by Formula $LiNi_{1-c2}M_{c2}O_2$ (where M is at least one selected from the group consisting of Co, Mn, Al, Cu, Fe, Mg, B and Ga, and $0.01 \leq c2 \leq 0.3$ is satisfied); lithium manganese composite oxides represented by Formula $LiMn_{2-c3}M_{c3}O_2$ (where M is at least one selected from the group consisting of Co, Ni, Fe, Cr, Zn, and Ta, and $0.01 \leq c3 \leq 0.1$ is satisfied) or $Li_2Mn_3MO_8$ (here, M is at least one selected from the group consisting of Fe, Co, Ni, Cu and Zn); lithium nickel cobalt manganese (NCM) composite oxides, lithium nickel cobalt manganese aluminum (NCMA) composite oxides, and $LiMn_2O_4$ in which a part of Li in the formula is substituted with alkaline earth metal ions, and the like, but is not limited thereto.

When the active material layer is a negative electrode active material layer, as the electrode active material, for example, a compound capable of reversible intercalation and deintercalation of lithium may be used. A specific example may include carbonaceous materials such as artificial graphite, natural graphite, graphitized carbon fiber, and amorphous carbon; metallic compounds capable of alloying with lithium, such as Si, Al, Sn, Pb, Zn, Bi, In, Mg, Ga, Cd, Si alloy, Sn alloy, or Al alloy; metal oxides capable of doping and de-doping lithium, such as $SiO_a$ ($0 < a < 2$), $SnO_2$, vanadium oxide, and lithium vanadium oxide; or a composite comprising the metallic compound and the carbonaceous materials such as a Si-C composite or a Sn-C composite, and the like, and any one or a mixture of two or more of the foregoing may be used.

A metal lithium thin film may also be used as the negative electrode active material, and as the carbon material, low crystalline carbon and high crystalline carbon, and the like may also be used. As the low crystalline carbon, soft carbon and hard carbon are representative, and as the high crystalline carbon, high-temperature baked carbons such as amorphous, plate-like, scale-like, spherical, or fibrous natural graphite or artificial graphite, Kish graphite, pyrolytic carbon, mesophase pitch based carbon fiber, mesocarbon microbeads, mesophase pitches, and petroleum and coal tar pitch derived cokes are representative.

The electrode active material may be included in the active material layer within a range of about 80 wt% to 99.5 wt%, or within a range of 88 wt% to 99 wt%, relative to the total weight of the active material layer, but the ratio may be changed depending on the use or design of the electrode.

The active material layer may further comprise a binder. The binder serves to improve attachment between the active materials, and adhesive force on between the active material layer and the current collector body. An example of the

binder is not particularly limited, and one or more may be selected from the group consisting of, for example, PVDF (poly(vinylidene fluoride), PVA (poly(vinyl alcohol)), SBR (styrene butadiene rubber), PEO (poly(ethylene oxide)), CMC (carboxyl methyl cellulose), cellulose acetate, cellulose acetate butylate, cellulose acetate propionate, cyanoethylpullulan, cyanoethyl polyvinyl alcohol, cyanoethyl cellulose, cyanoethyl sucrose, pullulan, polymethylmethacrylate, polybutylacrylate, polyacrylonitrile, polyvinylpyrrolidone, polyvinylacetate, an ethylene vinyl acetate copolymer (polyethylene-co-vinyl acetate), and polyarylate, and used.

In one example, the binder may be included in the active material layer within a range of 0.1 parts by weight to 10 parts by weight or 0.5 parts by weight to 5 parts by weight, relative to 100 parts by weight of the electrode active material, but is not limited thereto.

The active material layer may further comprise a conductive material, as needed. If the conductive material has conductivity without causing chemical changes in the secondary battery, it is not particularly limited, any known material can be used. For example, graphite such as natural graphite or artificial graphite; carbon black, such as carbon black, acetylene black, Ketjen black, channel black, furnace black, lamp black, or thermal black; conductive fibers such as carbon fibers or metal fibers; conductive tubes such as carbon nanotubes (CNTs); metal powders such as fluorocarbon, aluminum, or nickel powder; conductive whiskers such as zinc oxide or potassium titanate; conductive metal oxides such as titanium oxide; conductive materials such as polyphenylene derivatives, and the like may be used.

In one example, the conductive material may be included in the active material layer in an amount of 0.1 parts by weight to 20 parts by weight or 0.3 parts by weight to 10 parts by weight, relative to 100 parts by weight of the electrode active material, but is not limited thereto.

The active material layer may also further comprise any necessary known components in addition to the above-described components optionally

The method of forming the active material layer on the polymer layer to manufacture an electrode is not particularly limited. Typically, the active material layer is formed by coating a slurry containing the electrode active material, a binder, and a conductive material, and the like on the current collector (on the polymer layer), drying, and then rolling, and such a known method may be applied in the same manner.

The present specification also discloses an electrode assembly or an electrochemical element, for example, a secondary battery, which comprises such an electrode.

The electrochemical element may comprise the electrode as a positive electrode and/or a negative electrode. If the electrode of the present specification is used as a negative electrode and/or a positive electrode, other constitutions or manufacturing methods of the electrochemical element are not particularly limited, and known methods may be applied.

## Advantageous Effects

The present specification discloses a polymer layer, a production method thereof, and a use thereof. The polymer layer exhibits a so-called PTC (Positive Temperature Coefficient) effect at a required level at a required time, whereby it can be applied as a material that can secure stability in applications where stability problems due to abnormally high heat or flame are caused. The polymer layer can exhibit oxidation potential characteristics suitable for a use, and stably maintain such oxidation potential characteristics even under harsh environments. In the polymer layer, the PTC effect can rapidly appear at a desired level at a required time. The polymer layer has a flat surface, and thus has excellent resistance to scratches, and the like. The present specification discloses a production method and a use of the polymer layer.

## Description of Drawings

Figure 1 is a cross-sectional diagram of an exemplary current collector.
100: current collector body
200: polymer layer
Figure 2 is a cross-sectional diagram of an exemplary electrode.
100: current collector body
200: polymer layer
300: active material layer
Figure 3 is an NMR analysis result for a monomer of Preparation Example 2.

## Mode for Invention

Hereinafter, the polymer layer, and the like are specifically described through examples or comparative examples, but the scope of the polymer layer, and the like is not limited by the following examples.

1. NMR analysis

$^1$H-NMR analyses were performed at room temperature (about 25°C) using an NMR spectrometer having a 5 mm triple resonance probe and including a Bruker UltraShield spectrometer (300 MHz). A sample was diluted in a solvent ($CDCl_3$) for NMR measurement to a concentration of about 10 mg/ml or so and used, and the chemical shift was expressed in ppm.

2. GPC (Gel Permeation Chromatograph)

Molecular weight characteristics were measured using GPC (Gel permeation chromatography). A sample is placed in a 5 mL vial and diluted with chloroform to a concentration of about 1 mg/mL or so. The standard sample for calibration and the analysis sample were filtered through a syringe filter (pore size: 0.45 μm), and analyzed. As the analysis program, Waters' Empower 3 was used, and a weight average molecular weight (Mw) and a number average molecular weight (Mn) were each obtained by comparing the elution time of the sample with the calibration curve, and the molecular weight distribution (PDI) was calculated by the ratio (Mw/Mn). The measurement conditions of GPC are as follows.

<GPC measurement conditions>

Device: Waters' 2414
Column: using 3 Styragels from Waters
Solvent: THF (tetrahydrofuran)
Column temperature: 35°C
Sample concentration: 1mg/mL, 1μL injection
Standard sample: polystyrene (Mp: 3900000, 723000, 316500, 52200, 31400, 7200, 3940, 485)

3. Thickness measurement

A thickness of a polymer layer was measured using KEYENCE's VK-X series confocal laser microscope. An aluminum foil (current collector body) on which the polymer layer was formed was cut so that a width and a length were each 3 cm, thereby preparing a specimen, and about half of the polymer layer was removed from the specimen with acetone to expose the lower aluminum foil. The aluminum foil side of the specimen, where the polymer layer was not formed, was pressed against a flat plate, and measurement was started. An area where a width and a length were each 100 μm was observed under the microscope, but the polymer layer and the exposed aluminum foil of the specimen were each adjusted to be positioned approximately halfway within the observation area, and a 3D scan was performed. An average value P of heights was measured by optionally designating 10 points from the polymer layer within the observation area, and an average value A was obtained by measuring the heights optionally at 10 points on the aluminum foil, and then the value obtained by subtracting the average value A from the average value P was used as the thickness of the polymer layer.

4. Measurement of oxidation potentials $V_i$ and $V_f$

Oxidation potentials $V_i$ and $V_f$ were measured in the following manner. A polymer layer was formed on an aluminum foil (Al foil) (current collector body) having a thickness of about 15 μm. The method of forming the polymer layer and the thickness of the polymer layer were applied in the same manner as the contents described in each example and comparative example. A separator and a lithium film were laminated on the polymer layer to manufacture a laminate in which the aluminum foil/polymer layer/separator/lithium film were laminated, and the laminate was punched into a circle with a diameter of about 1.4 cm. A coin cell was manufactured by using a Wellcos CR2032 coin cell kit, and using the circularly punched laminate and an electrolyte. As the separator, the WL20C model from W Scope Korea was used; as the lithium film, a lithium film with a thickness of about 100 μm or so was used; and as the electrolyte, Enchem's product (1M $LiPF_6$ solution (solvent: EC/DMC/EMC =3/4/3 (mass ratio), EC: Ethylene Carbonate, DMC: dimethyl carbonate, EMC: ethylmethyl carbonate) was used. The oxidation potential of the coin cell was measured at about 25°C using an electrochemical meter (potentiostat) (Princeton Applied Research, PARASTAT-MC). The oxidation potential was measured by scanning the cyclic voltammetry (CV) at a scan rate of about 0.83 mV/sec in the range of 3V to 4.5V to the coin cell, and based on lithium and lithium ions ($Li/Li^+$). The oxidation potential measured after scanning the cyclic voltammetry once was designated as the oxidation potential $V_i$, and the oxidation potential measured after scanning 10 times was designated as the oxidation potential $V_f$.

5. AC impedance resistance at 3V and 3.3V

AC impedance resistance was measured to confirm electrical reactivity of a polymer layer.

Sample preparation

A separator and a lithium film were laminated on the polymer layer of the laminate of the aluminum foil (current collector body) and the polymer layer manufactured in examples or comparative examples to manufacture a laminate (aluminum foil/polymer layer/separator/lithium film), and the laminate was punched into a circle with a diameter of about 1.4 cm. A coin cell was manufactured by using the Wellcos CR2032 coin cell kit, and using the circularly punched laminate and an electrolyte. The separator was the WL20C model from W-SCOPE KOREA, as the lithium film, a lithium film with a thickness of 300 $\mu$m or so was used, and as the electrolyte, Enchem's product (1M $LiPF_6$ solution (solvent: EC/DMC/EMC =3/4/3 (mass ratio), EC: Ethylene Carbonate, DMC: dimethyl carbonate, EMC: ethylmethyl carbonate) was used.

External voltage 3V condition

AC impedance resistance of the manufactured coin cell was measured using an EIS (Electrochemical Impedance Spectronization) method. A voltage of 3V was applied to the coin cell at room temperature (25°C) for 10 minutes, and a Nyquist plot was obtained by the EIS measurement method in a range of 50,000 Hz to 0.1 Hz, and the interface resistance $R_{3V}$ obtained in the high frequency region of the Nyquist plot was measured. An electrochemical meter (potentiostat) (Princeton Applied Research, PARASTAT-MC) was used as the EIS measurement device.

External voltage 3.3V condition

A voltage of 3V was applied to the coin cell at room temperature (25°C) for 10 minutes, and, the AC impedance resistance $R_{3.3V}$ at the time point when 1 second passed after converting the applied voltage to 3.3V was measured in the same manner as the resistance $R_{3V}$.

6. AC impedance resistance at room temperature (25°C) and 130°C

(1) AC impedance resistance $R_{25}$ measurement

AC impedance resistance was measured using the same coin cell as in the measurement of the "5. AC impedance resistance at 3V and 3.3V" above. The AC impedance resistance of the coin cell was measured using the EIS (Electrochemical Impedance Spectronization) method. Specifically, a Nyquist plot was obtained using the EIS measurement method in the range of 50,000 Hz to 0.1 Hz, and the interface resistance obtained in the high frequency region from the obtained nyquist plot was measured. An electrochemical meter (potentiostat) (manufacturer: Princeton Applied Research, product name: PARASTAT-MC) was used as the EIS measurement device.

A voltage of 4.5V was applied to the coin cell at room temperature (25°C) for 10 minutes to maintain the doping state of the conductive polymer, and then the AC impedance resistance was measured after about 1 minute in a state where the external voltage was set to 0V (Open circuit voltage). The measured value was used as the AC impedance resistance $R_{25}$ of the polymer layer at room temperature (25°C).

(2) AC impedance resistance $R_{130}$ measurement

The coin cell was placed in the center of a convection oven (Jeotech, OF3-05W), and the coin cell was connected to an electrochemical meter (potentiostat) (Princeton Applied Research, PARASTAT-MC) outside the oven to enable EIS resistance measurement. While maintaining the room temperature (about 25°C) in this state, a voltage of 4.5V was applied to the coin cell (specimen) for 10 minutes to maintain the doping state of the conductive polymer, and the final temperature of the oven was set to 130°C. If the temperature reached 130°C, the AC impedance resistance was measured in the same manner as the resistance $R_{25}$ after about 1 minute in a state where the external voltage was set to 0V (Open circuit voltage). The measured value was used as the AC impedance resistance $R_{130}$ of the polymer layer at 130°C.

7. Average particle diameter

An average particle diameter (D50 particle diameter) of conductive particles was measured using a MASTERSIZER3000 device from Marvern in accordance with ISO-13320 standard. Toluene was used as a solvent upon measurement. If a sample is dispersed in the solvent and the laser is irradiated, the laser is scattered by the sample

dispersed in the solvent. Since the intensity and directionality of the scattered laser vary depending on the size of the particle, the average particle diameter can be obtained by analyzing this using a Mie theory. Through the above analysis, the measurement results were converted into particle diameters of spheres having the same volume as the dispersed sample to obtain a volume-based cumulative graph of particle size distribution, and the particle diameter (median particle diameter) at 50% accumulation of the graph was designated as the average particle diameter (D50 particle diameter).

8. Arithmetic average roughness Ra

Arithmetic average roughness Ra was measured using KEYENCE's VK-X series confocal laser microscope. An aluminum foil (current collector body) coated with a polymer layer was cut so that a width and a length were each 3 cm to prepare a specimen. The surface of the aluminum foil of the specimen, in which the polymer layer was not formed, was pressed against a flat plate and 3D scan was performed. The region of the polymer layer, where a width and a length were each 100μm was 3D scanned. After arbitrarily designating a region where a width and a length were each 50μm within the 3D scanned region, the arithmetic mean roughness Ra was measured using the 3D scan image.

9. Scratch resistance

A surface of a polymer layer was rubbed back and forth 10 times using dust-free fabric (Hansong Wiper MIRACLEAN322 Polyester) at a load of 100 g and a speed of 27 rpm. If scratches were confirmed upon observation with the naked eye, it was evaluated as NG, and if no scratches were observed upon observation with the naked eye, it was evaluated as PASS.

**Preparation Example 1. Polydopamine-coated conductive particles**

Carbon black particles (IMERYS, C-NERGY™ SUPER C65) were used as the conductive particles. The average particle diameter (D50 particle diameter) of the conductive particles was about 60 nm or so.

DHC (dopamine hydrochloride) (CAS No. 62-31-7) was added to a buffer solution and stirred at room temperature (about 25°C) to prepare a mixture. As the buffer solution, BIOSESANG's 0.1M pH 8.5 Tris-buffer product was used. The concentration of DHC in the mixture was about 2 mg/mL or so. The conductive particles were dispersed (sonication for 1 hour) in the mixture at a concentration of about 4 mg/mL or so, and stirred for additionally about 18 hours. As a result of the stirring, a polydopamine coating layer was formed on the surface of the conductive particles. After filtering under reduced pressure using a paper filter, the resultant particles were vacuum-dried to obtain conductive particles coated with polydopamine.

Preparation Example 2. Synthesis of monomer (A)

A monomer of Formula A below was synthesized in the following manner.

[Formula A]

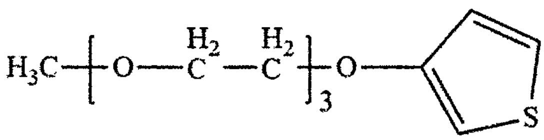

1.372 g (12.02 mmol, 1 eq) of 3-methoxythiophene and 3 g (16.83 mmol, 1.4 eq) of triethylene glycol monomethyl ether were dissolved in 100 ml of toluene together with 230 mg of p-toluenesulfonic acid (p-TsOH), and mixed. The mixture was refluxed at 120°C, and simultaneously reacted, whereby methanol produced by the reaction (transetherification) was removed by 4A type molecular sieves filled in an extractor (soxhlet extractor). The reactant was refluxed for 24 hours, quenched with water, extracted with ethyl acetate, and then washed with brine, and dried over magnesium sulfate ($MgSO_4$). The solvent was removed by a rotary evaporator, and the residue was purified by column chromatography eluting with methylene chloride/hexane (2:1) to obtain the target compound (monomer (A)). The NMR analysis results for the target compound (monomer (A)) are as shown in Figure 3.

**Preparation Example 3. Synthesis of conductive polymer (A)**

To a solution in which 3.20 g (19.71 mmol, 3 eq) of iron (III) chloride was dissolved in 150 ml of methylene chloride. 0.7 g (2.96 mmol, 0.6 eq) of 3-dodecylthiophene, 0.3 g (1.48 mmol, 0.3 eq) of 3-hexylthiophene, 0.12 g (0.49 mmol, 0.1 eq)

of the monomer (A) of Preparation Example 2, and 0.37 g (1.23 mmol, 0.3 eq) of pyrrole were introduced, and polymerized at 25°C for 24 hours to prepare a conductive polymer (A). In the conductive polymer (A), the molar ratio of the 3-dodecylthiophene unit (I), the 3-hexylthiophene unit (II), the monomer (A) unit (III) of Preparation Example 2, and the pyrrole (IV) unit was 2.96:1.48:0.49:1.23 (I: II: III: IV) or so. The polymerization solution was placed in an osmosis membrane with an MWCO (molecular weight of cut-off) of 5000, and then immersed in 200ml of an acetonitrile solvent to remove unreacted iron chloride and monomers. The residue precipitated inside the osmosis membrane was washed with methanol, and dried at 60°C for 12 hours to obtain a conductive polymer (A). The weight average molecular weight (Mw) and number average molecular weight (Mn) of the conductive polymer (A) were 99,000 g/mol and 28,000 g/mol or so, respectively.

**Example 1.**

A mixture obtained by mixing the conductive polymer (A) of Preparation Example 3 and the polydopamine-coated conductive particles (P) of Preparation Example 1 in a weight ratio (A: P) of 70:30 was dispersed in toluene at a concentration of about 4 wt% or so to prepare a polymer solution. Upon preparing the polymer solution, the mixture was dispersed at a temperature of about 30°C or so for about 4 hours using an ultrasonic disperser. The polymer solution was coated on the current collector body using a Meyer bar coating method. Subsequently, the coating layer was maintained in a drying oven at a temperature of about 140°C or so for about 4 minutes or so (primary heat treatment). Thereafter, the current collector body on which the coating layer was formed was placed in an oven and heat-treated at a temperature of about 110°C or so for 18 hours or so (secondary heat treatment) to form a polymer layer having a thickness of about 400 nm. As the current collector body, an Al foil having a thickness of about 15 μm or so was used.

**Example 2.**

A polymer layer was formed in the same manner as in Example 1, except that a mixture obtained by mixing the conductive polymer (A) of Preparation Example 3 and the polydopamine-coated conductive particles (P) of Preparation Example 1 int a weight ratio of 60:40 (A: P) was used upon preparing the polymer solution.

**Example 3.**

A polymer layer was formed in the same manner as in Example 1, except that a mixture obtained by mixing the conductive polymer (A) of Preparation Example 3 and the polydopamine-coated conductive particles (P) of Preparation Example 1 int a weight ratio of 50:50 (A: P) was used upon preparing the polymer solution.

**Comparative Example 1.**

A polymer solution was prepared by dispersing the conductive polymer (A) of Preparation Example 3 in toluene at a concentration of about 4 wt% or so. Upon preparing the polymer solution, the conductive polymer was dispersed at a temperature of about 30°C for about 4 hours using an ultrasonic disperser. The polymer solution was coated on the current collector body using a Meyer bar coating method, and dried in a drying oven at about 140°C for about 4 minutes or so to form a polymer layer having a thickness of about 400 nm or so. As the current collector body, an Al foil having a thickness of about 15 μm or so was used.

**Comparative Example 2.**

Carbon black particles (IMERYS, C NERGYTM SUPER C65) without coating with polydopamine were used as conductive particles. A polymer solution and a polymer layer were formed in the same manner as in Example 1 using a mixture obtained by mixing the conductive polymer (A) of Preparation Example 3 and the carbon black particles (C) in a weight ratio of 90:10 (A: P).

**Comparative Example 3.**

As conductive particles, carbon black particles (IMERYS, C NERGYTM SUPER C65) without coating with polydopamine were used. A polymer solution and a polymer layer were formed in the same manner as in Example 1 using a mixture obtained by mixing the conductive polymer (A) of Preparation Example 3 and the carbon black particles (C) in a weight ratio (A: P) of 80:20.

**Comparative Example 4.**

As conductive particles, carbon black particles (IMERYS, C NERGYTM SUPER C65) without coating with polydopamine were used. A polymer solution was prepared in the same manner as in Example 1 using a mixture obtained by mixing the conductive polymer (A) of Preparation Example 3 and the carbon black particles (C) in a weight ratio (A: P) of 10:4. Subsequently, a polymer layer was formed in the same manner as in Example 1 using the polymer solution, but a polymer layer was formed by performing only primary heat treatment without performing secondary heat treatment.

Table 1 summarizes the oxidation potentials $V_i$ and $V_f$ measured in the "4. Measurement of oxidation potentials $V_i$ and $V_f$" section, and Δ calculated by substituting the oxidation potentials into the equation $100\times(V_f-V_i)/V_i$. In Table 1, the units of the oxidation potentials $V_i$ and $V_f$ are V, and the unit of ΔV is %.

[Table 1]

| | Example | | | Comparative Example | | | |
|---|---|---|---|---|---|---|---|
| | 1 | 2 | 3 | 1 | 2 | 3 | 4 |
| Vi | 3.42 | 3.41 | 3.37 | 3.6 | 3.5 | 3.45 | 3.57 |
| Vf | 3.45 | 3.43 | 3.4 | 3.9 | 3.73 | 3.6 | 3.71 |
| ΔV | 0.88 | 0.59 | 0.89 | 8.33 | 6.57 | 4.35 | 3.92 |

From Table 1, it can be confirmed that the polymer layers of Examples exhibit a low oxidation potential $V_i$, and such a low oxidation potential is stably maintained even after repeated cyclic voltammetry scans.

Table 2 summarizes the results of $R_{3V}$, $R_{3.3V}$, $R_{25}$, and $R_{130}$ measured in the measurement sections of "5. AC impedance resistance at 3V and 3.3V" and "6. AC impedance resistance at room temperature (25°C) and 130°C" above. In Table 2, Q is the value ($R_{3V}/R_{3.3V}$) obtained by dividing $R_{3V}$ by $R_{3.3V}$, and P is the value ($R_{130}/R_{25}$) obtained by dividing $R_{130}$ by R25.

In Table 2, the units of $R_{3V}$, $R_{3.3V}$, $R_{25}$, and $R_{130}$ are Ω.

[Table 2]

| | Example | | | Comparative Example | | | |
|---|---|---|---|---|---|---|---|
| | 1 | 2 | 3 | 1 | 2 | 3 | 4 |
| $R_{3V}$ | 39,000 | 38,000 | 35,000 | 40,000 | 35,000 | 35,000 | 38,000 |
| $R_{3.3V}$ | 400 | 350 | 250 | 35,000 | 1,800 | 400 | 1,000 |
| Q | 97.5 | 108.6 | 140 | 1.1 | 20 | 87.5 | 38 |
| $R_{25}$ | 40 | 33 | 22 | 800 | 60 | 45 | 50 |
| $R_{130}$ | 34,000 | 32,000 | 33,000 | 40,000 | 30,000 | 30,000 | 40,000 |
| P | 850 | 969.7 | 1,500 | 50 | 500 | 666.7 | 800 |

From the results of Table 2, it can be known that the polymer layers of Examples show a rapid decrease in impedance immediately after the external voltage changes from 3V to 3.3V. This means that the electrical reactivity of the doping/de-doping of the polymer layer is very fast. In addition, it can be confirmed that the increase in resistance occurs efficiently depending on the temperature change, thereby showing a high P value.

Table 3 summarizes the evaluation results of "8. Arithmetic mean roughness Ra" and "9. Scratch resistance" together with the thickness of each polymer layer. In Table 3, the units of thickness and Ra are nm.

[Table 3]

| | Example | | | Comparative Example | | | |
|---|---|---|---|---|---|---|---|
| | 1 | 2 | 3 | 1 | 2 | 3 | 4 |
| Thickness | 400 | 400 | 400 | 400 | 400 | 400 | 400 |
| Ra | 140 | 150 | 160 | 50 | 220 | 250 | 300 |
| Ra/Thickness | 0.35 | 0.375 | 0.4 | 0.125 | 0.55 | 0.625 | 0.75 |

(continued)

| | Example | | | Comparative Example | | | |
|---|---|---|---|---|---|---|---|
| | 1 | 2 | 3 | 1 | 2 | 3 | 4 |
| Scratch resistance | PASS | PASS | PASS | NG | NG | NG | PASS |

From the results of Table 3, it can be confirmed that the polymer layers of Examples show a low Ra even when it contains conductive particles at the same level as Comparative Examples, whereby the scratch resistance is stably maintained.

## Claims

1. A polymer layer comprising a conductive polymer and a conductive material, wherein

an oxidation potential is 4 V or less, and
an absolute value of ΔV of Equation 1 below is 3% or less:

[Equation 1]

$$\Delta V = 100 \times (V_f - V_i)/V_i$$

wherein, $V_i$ is the oxidation potential of the polymer layer based on $Li/Li^+$ after one cyclic voltammetry scan, $V_f$ is the oxidation potential of the polymer layer based on $Li/Li^+$ after 10 cyclic voltammetry scans, and the cyclic voltammetry scan is performed at a scan rate of 0.83 mV/sec in a range of 3V to 4.5V.

2. The polymer layer according to claim 1, wherein Q of Equation 2 below is 10 or more:

[Equation 2]

$$Q = R_{3V}/R_{3.3V}$$

wherein, $R_{3V}$ is the AC impedance resistance of the polymer layer at 25°C and 3V, and $R_{3.3V}$ is the AC impedance resistance of the polymer layer at the time point when the 25°C and 3V conditions have been changed to 25°C and 3.3V conditions, and 1 second has passed.

3. The polymer layer according to claim 1, having arithmetic mean roughness Ra of 200 nm or less.

4. The polymer layer according to claim 1, wherein a ratio Ra/T of arithmetic mean roughness Ra of the polymer layer to a thickness T of the polymer layer is 0.5 or less.

5. The polymer layer according to claim 1, wherein P by Equation 3 below is 60 or more:

[Equation 3]

$$P = R_{130}/R_{25}$$

wherein, $R_{25}$ is the AC impedance resistance of the polymer layer at 25°C, and $R_{130}$ is the AC impedance resistance of the polymer layer at 130°C.

6. The polymer layer according to claim 1, wherein the conductive polymer has a long-chain hydrocarbon functional group.

7. The polymer layer according to claim 6, wherein the conductive polymer contains, as the long-chain hydrocarbon

functional group, a first hydrocarbon functional group with 10 or more carbon atoms and a second hydrocarbon functional group with 9 or less carbon atoms.

**8.** The polymer layer according to claim 1, comprising a functional group of Formula 1 below:

[Formula 1]

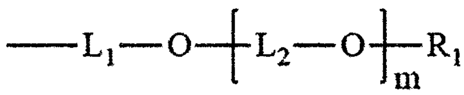

wherein, $L_1$ is a single bond, an alkylene group or an alkylidene group, $L_2$ is an alkylene group or an alkylidene group, $R_1$ is hydrogen or an alkyl group, and m is a number in a range of 1 to 10.

**9.** The polymer layer according to claim 1, wherein the conductive polymer contains a thiophene monomer unit and a no-thiophene monomer unit.

**10.** The polymer layer according to claim 9, wherein the no-thiophene monomer unit is an aromatic monomer unit or a nitrogen-containing heterocyclic monomer unit.

**11.** The polymer layer according to claim 9, wherein a molar ratio of the thiophene monomer unit in the conductive polymer is 70 mol% or more, and 0.05 mol or more of the no-thiophene monomer unit exists per 1 mol of the thiophene monomer unit.

**12.** The polymer layer according to claim 9, wherein the no-thiophene monomer unit is at least one unit selected from the group consisting of units of Formulas 10 to 15 below:

[Formula 10]

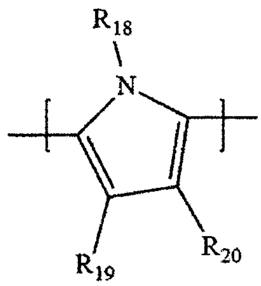

wherein, $R_{18}$, $R_{19}$, and $R_{20}$ are each independently hydrogen, a polar functional group, or a hydrocarbon functional group:

[Formula 11]

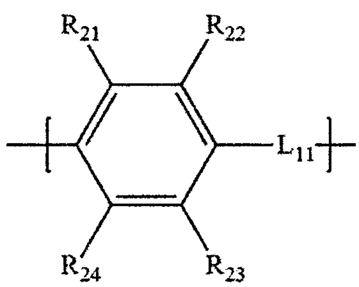

wherein, $L_{11}$ is a single bond, an alkylene group, an alkylidene group, O, or $NR^1$, where $R^1$ above is hydrogen, an alkyl group, an alkenyl group, or an aryl group:

[Formula 12]

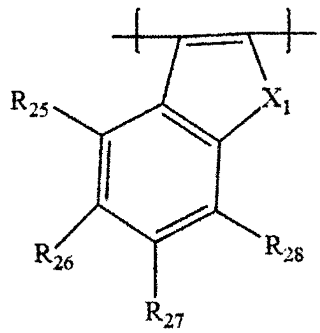

wherein, $X_1$ is S, O, or $NR^1$, where $R_1$ is hydrogen, an alkyl group, an alkenyl group, or an aryl group, and $R_{25}$ to $R_{28}$ are each independently hydrogen, an alkyl group, an alkenyl group, an alkoxy group, an aryl group, or halogen:

[Formula 13]

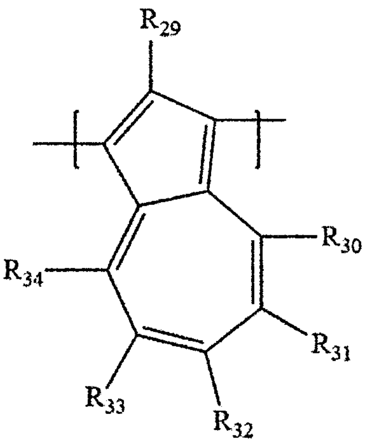

wherein, $R_{29}$ to $R_{34}$ are each independently hydrogen, an alkyl group, an alkenyl group, an alkoxy group, an aryl group, a hydroxy group, or halogen:

[Formula 14]

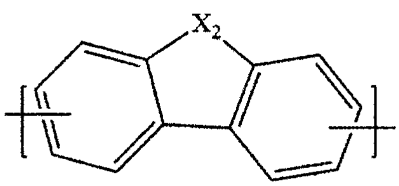

wherein, $X_2$ is $CR^aR^b$ or $NR^1$, where $R^a$, $R^b$ and $R^1$ above are each independently hydrogen, an alkyl group, an alkenyl group or an aryl group, and the unit of Formula 14 above is unsubstituted or substituted with one or more selected from the group consisting of an alkyl group, an alkenyl group, an alkoxy group, an aryl group, and halogen:

[Formula 15]

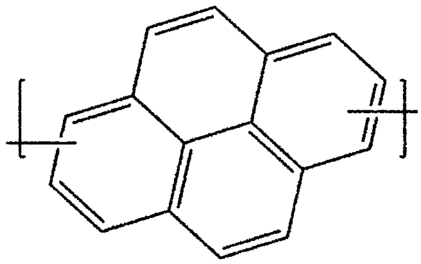

wherein, the unit of Formula 15 is unsubstituted or substituted with one or more selected from the group consisting of an alkyl group, an alkenyl group, an alkoxy group, an aryl group, and halogen.

**13.** The polymer layer according to claim 1, wherein the conductive material is at least one selected from the group consisting of carbon particles, carbon fibers, graphene, graphite, carbon black, carbon nanotubes, and metal particles.

**14.** The polymer layer according to claim 1, wherein the conductive material is surface-treated with a polyphenol-based compound.

**15.** A production method of a polymer layer comprising:

a first step of performing primary heat treatment on a polymer layer precursor including a conductive polymer and a conductive material; and
a second step of performing secondary heat treatment on the polymer layer precursor undergone the first step, wherein
a ratio $T_1/T_2$ of the heat treatment temperature $T_1$ of the primary heat treatment to the heat treatment temperature $T_2$ of the secondary heat treatment is 0.1 or more.

**16.** The production method of a polymer layer according to claim 15, wherein a ratio $S_2/S_1$ of the heat treatment time $S_2$ of the secondary heat treatment to the heat treatment time $S_1$ of the primary heat treatment is 50 or more.

**17.** A current collector comprising:

a current collector body; and
the polymer layer of any one of claims 1 to 14 formed on one or both sides of the current collector body.

**18.** An electrode comprising:

a current collector body;
the polymer layer of any one of claims 1 to 14 formed on one or both sides of the current collector body; and
an active material layer formed on the polymer layer.

**19.** An electrode assembly comprising the electrode of claim 18.

**20.** A secondary battery comprising the electrode assembly of claim 19.

[Figure 1]

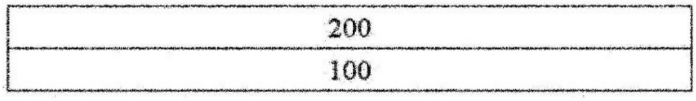


[Figure 2]

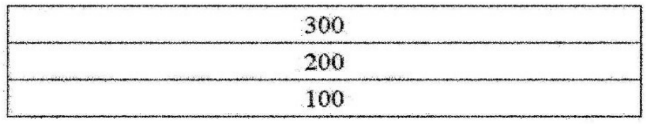


[Figure 3]

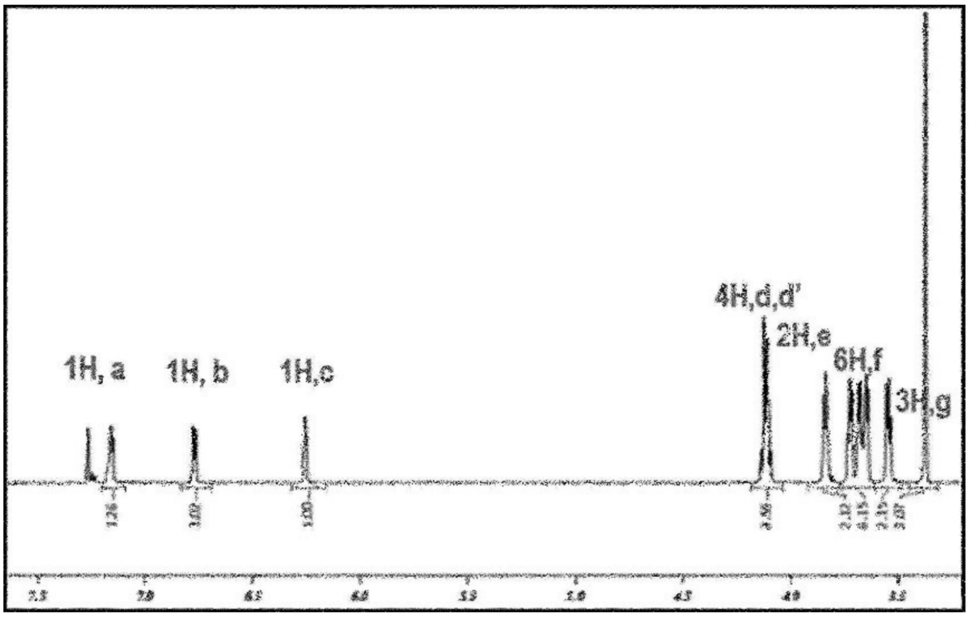

**INTERNATIONAL SEARCH REPORT**

International application No. **PCT/KR2024/012244**

**A. CLASSIFICATION OF SUBJECT MATTER**

**H01M 4/66**(2006.01)i; **H01M 4/13**(2010.01)i; **H01M 4/139**(2010.01)i; **H01M 4/04**(2006.01)i; **H01M 10/052**(2010.01)i; **H01M 10/42**(2006.01)i; **H01M 4/62**(2006.01)i; **H01M 4/02**(2006.01)i

According to International Patent Classification (IPC) or to both national classification and IPC

**B. FIELDS SEARCHED**

Minimum documentation searched (classification system followed by classification symbols)

H01M 4/66(2006.01); B05D 3/00(2006.01); C09D 5/24(2006.01); H01M 10/052(2010.01); H01M 10/42(2006.01); H01M 4/04(2006.01); H01M 4/13(2010.01); H01M 4/134(2010.01); H01M 50/486(2021.01); H01M 50/581(2021.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 집전체(collector), 전도성 고분자(conductive polymer), 도전성 물질(conductive material), 티오펜(thiophene), 고분자층(polymer layer), 표면 처리(surface treatment), 폴리페놀(polyphenol), 산화전위(oxidation potential), 이차 전지(secondary battery)

**C. DOCUMENTS CONSIDERED TO BE RELEVANT**

| Category* | Citation of document, with indication, where appropriate, of the relevant passages | Relevant to claim No. |
|---|---|---|
| Y<br>A | CN 112467314 A (DONGGUAN CHAM BATTERY TECHNOLOGY CO., LTD.) 09 March 2021 (2021-03-09)<br>See claims 8 and 10; and example 1. | 15,16<br>1-14,17-20 |
| Y | KR 10-2104974 B1 (LG DISPLAY CO., LTD.) 27 April 2020 (2020-04-27)<br>See claim 1; and paragraphs [0056] and [0059]. | 15,16 |
| A | KR 10-2022-0043773 A (LG ENERGY SOLUTION, LTD.) 05 April 2022 (2022-04-05)<br>See claims 1-18; and paragraph [0074]. | 1-20 |
| A | US 2023-0231276 A1 (AMIONX, INC.) 20 July 2023 (2023-07-20)<br>See claims 1, 2, 5, 7 and 12. | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **02 December 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/012244**

**C. DOCUMENTS CONSIDERED TO BE RELEVANT**

| Category* | Citation of document, with indication, where appropriate, of the relevant passages | Relevant to claim No. |
|---|---|---|
| A | KR 10-2023-0037451 A (LG ENERGY SOLUTION, LTD.) 16 March 2023 (2023-03-16)<br>See claims 1-9. | 1-20 |

Form PCT/ISA/210 (second sheet) (July 2022)

**INTERNATIONAL SEARCH REPORT**
**Information on patent family members**

International application No.
**PCT/KR2024/012244**

| Patent document cited in search report | Publication date (day/month/year) | Patent family member(s) | Publication date (day/month/year) |
|---|---|---|---|
| CN 112467314 A | 09 March 2021 | None | |
| KR 10-2104974 B1 | 27 April 2020 | KR 10-2015-0026067 A | 11 March 2015 |
| KR 10-2022-0043773 A | 05 April 2022 | CN 116420247 A | 11 July 2023 |
| | | EP 4203094 A1 | 28 June 2023 |
| | | US 2023-0335709 A1 | 19 October 2023 |
| | | WO 2022-071704 A1 | 07 April 2022 |
| US 2023-0231276 A1 | 20 July 2023 | CN 116470245 A | 21 July 2023 |
| | | EP 4213272 A1 | 19 July 2023 |
| | | JP 2023-104917 A | 28 July 2023 |
| | | KR 10-2023-0111591 A | 25 July 2023 |
| KR 10-2023-0037451 A | 16 March 2023 | CN 116918093 A | 20 October 2023 |
| | | EP 4293742 A1 | 20 December 2023 |
| | | JP 2024-509209 A | 29 February 2024 |
| | | US 2024-0162446 A1 | 16 May 2024 |
| | | WO 2023-038474 A1 | 16 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230108457
- KR 1020230108421
- KR 1020230115816